Europäisches Patentamt

European Patent Office

Office européen des brevets

(11) Veröffentlichungsnummer : **0 468 023 B1**

(19)

## (12) EUROPÄISCHE PATENTSCHRIFT

(45) Veröffentlichungstag der Patentschrift :
18.10.95 Patentblatt 95/42

(21) Anmeldenummer : **91903579.0**

(22) Anmeldetag : **08.02.91**

(86) Internationale Anmeldenummer :
**PCT/EP91/00241**

(87) Internationale Veröffentlichungsnummer :
**WO 91/12518 22.08.91 Gazette 91/19**

(51) Int. Cl.⁶ : **G01N 22/04**

(54) VERFAHREN ZUR MESSUNG DER MATERIALFEUCHTE EINES MESSGUTES MIT HILFE VON MIKROWELLEN UND VORRICHTUNG ZUR DURCHFÜHRUNG DES VERFAHRENS.

(30) Priorität : **10.02.90 DE 4004119**

(43) Veröffentlichungstag der Anmeldung :
**29.01.92 Patentblatt 92/05**

(45) Bekanntmachung des Hinweises auf die
Patenterteilung :
**18.10.95 Patentblatt 95/42**

(84) Benannte Vertragsstaaten :
**AT CH DE ES FR GB IT LI SE**

(56) Entgegenhaltungen :
**EP-A- 0 372 992
DE-A- 2 848 993
DE-B- 2 340 130
US-A- 4 155 035**

(73) Patentinhaber : **TEWS ELEKTRONIK
Sperberhorst 10
D-22459 Hamburg (DE)**

(72) Erfinder : **TEWS, Manfred
Sperberhorst
D-2000 Hamburg 61 (DE)**
Erfinder : **SIKORA, Jan
Brödermannsweg 72
D-2000 Hamburg 61 (DE)**
Erfinder : **HERRMANN, Rainer
Kottwitzstrasse 15
D-2000 Hamburg 20 (DE)**

(74) Vertreter : **Schmidt-Bogatzky, Jürgen, Dr. Ing.
Warburgstrasse 50
D-20354 Hamburg (DE)**

EP 0 468 023 B1

## Beschreibung

Die Erfindung betrifft ein Verfahren zur Messung der Materialfeuchte eines Meßgutes mit Hilfe von Mikrowellen, bei dem durch einen Prozessor gesteuert in einem Mikrowellen-Generator eine elektromagnetische Strahlung variabler Frequenz erzeugt und einen als Resonator ausgebildeten Probenapplikator zugeführt wird, bei dem das aus dem Applikator austretende Mikrowellensignal einer Detektor-Diode zugeführt wird, aus deren Signalen über einen Analog/Digital-Wandler vom Rechner als primäre Meßgrößen b(0) und f(0) ermittelt werden, wobei b(0) die Halbwertsbreite bei der Resonanzfrequenz f(0) des mit einer Meßprobe in Wirkverbindung stehenden Applikators ist, und eine Vorrichtung zur Durchführung des Verfahrens.

In zahlreichen industriellen Verfahrensprozessen ist die genaue Kenntnis des Feuchtegehaltes der dem Verfahrensprozeß unterworfenen Materialien von großer Bedeutung. Nur wenn die exakte Information über die Materialfeuchte der Schüttgüter, Lebensmittel-Produkte, Pasten usw. sofort vorliegt, kann regelnd auf die Parameter des Produktionsprozesses eingegriffen werden. In vielen Fällen hängt davon direkt die gewünschte Verbesserung der Produktqualität, der Produkthaltbarkeit und der energetisch optimalen Nutzung der Prozessanlage ab.

Es sind daher schon die verschiedensten Meßmethoden zur Ermittlung der Feuchte von Stoffen vorgeschlagen worden, die auf Leitfähigkeitsmessung, kapazitiver Messung, Infrarotmessung, der Messung nach dem Neutronenstreuverfahren und dergleichen beruhen. Diese Verfahren haben jedoch die verschiedensten Nachteile, die ihre Einsatzfähigkeit einschränken.

Bekannt sind auch verschiedene Verfahren, bei denen mit Hilfe von Mikrowellen die Feuchte eines Stoffes ermittelt werden soll. Diese basieren entweder als Reflektions-, Durchstrahlungs- oder Resonanzverfahren auf der großen Dielektrizitätskonstanten und dem großen Verlustfaktor des Wassers. Resonanzverfahren sind auch zur Messung der Feuchte eines Meßgutes mit Hilfe von Mikrowellen bekannt. Hierbei wird das als solches bekannte Meßgut in einem hinsichtlich seiner mechanischen und elektrischen Eigenschaften bekannten Applikator vermessen, der als Resonator ausgebildet ist. Voraussetzung für die Messung ist die Aufnahme einer Kalibrationskurve, wozu das Meßgut bei verschiedenen bekannten Feuchtegraden ausgemessen wird. Der Nachteil dieser bekannten Mikrowellenverfahren besteht darin, daß sie meist gleichzeitig zur Feuchtemessung eine Messung der Material-Dichte mit anderen Meßverfahren wie Wiegen und dergleichen erfordern, um die störende Beeinflussung der Mikrowellensignale durch Dichteschwankungen auszuschließen. Bisher bekannte dichteunabhängige Mikrowellen-Feuchtemeßverfahren weisen den großen Nachteil auf, daß sie nur bei einer begrenzten Anzahl von Materialien und in einem engen Bereich der Materialfeuchte einsetzbar sind, Vor- und Nachkalibrationen erfordern oder nicht vor Ort unter Prozeßbedingungen kalibriert werden können. Daher ergeben sich bei bisher bekannten Verfahren für den Einsatz zur Feuchtemessung bei industriellen Prozessen große Beschränkungen.

Ein Verfahren der eingangs genannten Art ist in der ZS "Microwave Power", Vol. 15, No. 8, 1989, S. 648 - 649 beschrieben. Bei diesem Verfahren besteht jedoch der Nachteil, daß die Generatorfrequenz nicht einstellbar ist. Es muß eine Kurve in dem Rechner abgelegt werden, die die Steuerspannung des VCO mit der Mikrowellen-Frequenz des VCO verbindet. Dies bewirkt aber nur eine grobe Verknüpfung von Generatorfrequenzen und Steuerspannung, da diese Beziehung ständigen Veränderungen durch Generatorbelastung, Temperatur, Alterung u. dgl. unterworfen ist. Nach diesem Verfahren können die Resonanzfrequenz-Änderungen und Breite-Änderungen nur sehr grob erfaßt werden. Dies hat zur Folge, daß eine langzeitstabile Kalibration des Gerätes mit Verknüpfung des Meßsignals A und der Materialfeuchte oder eine einigermaßen genaue Erfassung der Resonanzfrequenzen und Breiten nicht möglich ist. Weiter ist dieses Verfahren nur für die Vermessung von Materialproben niedriger Feuchtwerte geeignet. Ursache hierfür ist, daß zur Erlangung einer gewissen Trennung von Feuchte - und Dichteeinfluß ein Ausdruck verwendet wird, wie er bereits durch die DE-A-29 28 487 bekannt war. Dieses Verfahren ist allenfalls geeignet, in einigen speziellen Anwendungsfällen niedriger Feuchtewerte auch dichteunabhängig messen zu können, solange die Dichteschwankungen nur im Bereich kleiner Dichtewerte bleiben, wobei nur niedrige Resonatorstörungen auftreten. Will man dagegen größere Resonatorstörungen erfassen, muß auch die exakte Messung von Resonanzfrequenz und Breite möglich sein, was nach dem bekannten Verfahren unmöglich ist.

In der ZS "G-I-T Fachzeitschrift für das Laboratorium", 18. Jg. Sept. 1974, wird ferner eine Meßanordnung beschrieben, mittels der durch Veränderung der Steuerspannung des Mikrowellengenerators (VCO) die Mikrowellen-Frequenz in einem großen Bereich variiert wird, ohne die genaue Beziehung zwischen Steuerspannung und Generatorfrequenz zu kennen, die von Alter, Sorte, Ausgangslast und Temperatur des Mikrowellengenerators abhängig ist. Mit der Detektor-Diode wird deshalb das Resonator-Ausgangssignal auch nur auf den Maximalwert abgetastet, der beim Durchfahren der Resonanzfrequenz erreicht wird. Damit kann dieses Gerät nur die Resonanzamplitude des gefüllten Resonators im Vergleich zur Resonanzamplitude des leeren Resonators messen. Diese Dämpfungsmessung ist aber genauso empfindlich auf die Packungsdichte wie auf die Materialfeuchte. Deshalb kann mit diesem Verfahren nur dort die Materialfeuchte gemessen werden, wo die Packungsdichte konstant bleibt. Feuchte- und Dichteänderungen können mit dieser Methode nicht auseinandergehalten werden. Dieses Gerät hat sich aus diesem Grunde im praktischen Einsatz nicht durchgesetzt.

Die Aufgabe der Erfindung besteht darin, ein Verfahren der eingangs genannten Art so zu verbessern, daß mittels einer hierzu geeigneten Vorrichtung die Materialfeuchte in industriellen Prozessen mit großer Genauigkeit gemessen werden kann, ohne daß eine meßtechnische Abhängigkeit von der Dichte des Materials oder der Materialsorte besteht, wobei gleichzeitig auch unabhängig von dem jeweiligen Feuchtegehalt die Dichte des Materials durch Auswertung des Mikrowellensignals meßbar sein soll. Gleichzeitig soll der Einfluß der Materialsorte, deren Feuchte bestimmt werden soll, auf das Mikrowellensignal so verkleinert werden, daß bei Schwankungen in der chemischen Zusammensetzung des Materials keine neue Kalibrationskurve aufgenommen werden muß.

Erfindungsgemäß erfolgt die Lösung der Aufgabe bezüglich des Verfahrens durch die kennzeichnenden Merkmale des Anspruchs 1 und 2 und bezüglich der Vorrichtung durch die kennzeichnenden Merkmale des Anspruchs 9. Vorteilhafte Ausgestaltungen der Erfindung werden in den abhängigen Ansprüchen beschrieben.

Nach dem erfindungsgemäßen Verfahren ist es möglich, die Materialfeuchte mit Hilfe eines Mikrowellen-Resonator-Verfahrens im gesamten in industriellen Prozessen vorkommenden Feuchtebereichen messen zu können. Das gleiche Meßgerät kann ohne irgendeine Hardware-Umstellung einmal im PPM-Bereich und das nächste Mal bis zu Feuchtewerten über 80% eingesetzt werden. Bei jedem Meßfall hat die Dichte im Bereich lockerer Schüttung bis zu maximaler Pressung keinen Einfluß. An die Probenform werden keine besonderen Anforderungen gestellt.

Die Resonanzbreite wird über die Resonanzfrequenz in Abhängigkeit von der Packungsdichte berücksichtigt. Das Meßsignal $\Phi$ ist also die Steigung der Geraden, die die Resonanzbreite-Frequenz-Meßpunkte beschreibt, wenn die Packungsdichte, nicht aber die Feuchte des Materials im Resonator verändert wird. Das feuchteabhängige Meßsignal $\Phi$ kann auch gemessen werden, ohne die Eigenschaften des leeren Resonators zu kennen. Hierzu werden die Breite-Frequenz-Werte in verschiedenen Dichtezuständen einer Meßprobe im Resonator gemessen und gespeichert. Dann wird die Regressionsgerade rechnerisch ermittelt, der Wert der Steigung der Geraden des feuchteabhängigen Meßwertes festgehalten und über die Kalibrationskurve der Feuchtewert bestimmt. Diese Betriebsart des Gerätes ermöglicht Messungen, ohne daß der leere Resonator gemessen werden muß. Es sind lediglich Mehrfachmessungen am gleichen Produkt und gleicher Feuchte in unterschiedlichen Dichtezuständen erforderlich.

Dieses ist bei vielen komprimierbare Produkte gut möglich.

Es können auch Proben gemessen werden, bei denen die Geraden konstanter Feuchte oder unterschiedlicher Dichte sich nicht in einem Punkt schneiden. Denn wenn dagegen die Steigung $\Phi$ der Geraden konstanter Feuchte im Breite-Frequenz-Diagramm durch unterschiedliche Kompressionszustände ermittelt werden, bleibt das Meßsignal $\Phi$ streng dichteunabhängig.

Gegenüber bekannten Mikrowellen-Meßverfahren kann das erfindungsgemäße Verfahren auch dann eingesetzt werden, wenn sich der Leerzustand des Resonators ständig ändert, wie z. B. bei starken Verschmutzungen durch das Produkt. Leer-Resonanzmessungen sind ferner nicht möglich, wenn das Produkt in einem Strang verläuft.

Der Einsatz der erfindungsgemäßen Vorrichtung kann im Probe-Entnahme-Verfahren oder direkt im industriellen Prozeß erfolgen, wobei sich das Meßverfahren durch folgende Vorteile auszeichnet:

- Die Reproduzierbarkeit und Genauigkeit der Messung am unveränderten Produkt ist gesteigert. Die Grenze der Meßgenauigkeit wird so allein durch die Qualität der Vergleichsmessung begründet, die dem Meßverfahren in Form einer Kalibrationskurve zugrunde liegt.
- Die Aufnahme einer Kalibrationskurve ist direkt im Prozeß, unter den Bedingungen des Einsatzes der Vorrichtung mit einfachster Bedienung möglich. Eine Vor- oder Nachkalibration entfällt.
- Nach Beginn der Messung steht das Meßergebnis in kürzest möglicher Zeit für die Prozeßsteuerung zur Verfügung (Meßzeit in der Regel etwa 1 s).
- Eine Veränderung der Materialdichte bei gleichbleibender Material-Feuchte - etwa durch Pressung oder Quellung des Materials, oder durch Veränderung der Korngröße, usw. - hat keinen Einfluß auf das Meßsignal. Das Meßverfahren ist dichte-unabhängig, es hängt nur von der Material-Feuchte ab. Damit ist auch ein gesondertes Verfahren zur Dichtemessung überflüssig, wie es bei gattungsgemäßen Vorrichtungen üblich ist, z. B. durch gleichzeitiges Wiegen des Meßgutes, durch Anforderung eines definierten Füll-Volumens oder durch radiometrische Methoden.
- Eine Veränderung der Sorte des Meßgutes, d. h. Veränderung des Meßgutes bei gleichbleibender Grundstruktur wie z. B. Wechsel der Tabak-Sorte, Kaffee-Sorte, Milch-Produkt-Sorte usw. wirkt sich für die Feuchtemessung nicht aus, da die Kalibrations-Kurve nur unwesentlich beeinflußt wird. Damit kann eine einmal aufgenommene Kalibrationskurve auch bei Veränderung der Material-Sorte beibehalten werden, solange die Grundstruktur des Materials erhalten bleibt.

Dies ist eine Folge der besonderen Signalverarbeitung und der Mikrowellen-Meßtechnik, wo der im niederfrequenten Bereich störende Einfluß der ionischen Leitfähigkeit von untergeordneter Bedeutung ist. Beim Wechsel des Materials anderer Grundstruktur können die unterschiedlichen Kalibrationskurven abgespeichert und im Bedarfsfall abgerufen werden.

- Durch die passende Wahl des Frequenzbereiches des Meßverfahrens sowie die besondere Anpassung des Applikators als Meßaufnehmer an das Meßgut wirken sich Inhomogenitäten im Bereich des erfaßten Materials nicht störend aus.

Die Materialfeuchte wird als Mittelwert gemessen, im Gegensatz zu Meßverfahren, die nur eine kleine, aktive Meßzone besitzen wie z. B. Infrarot-Verfahren oder Mikrowellen-Verfahren mit Wellenlängen unterhalb der Größe der Kornstrukturen des Meßgutes.

- Besondere Oberflächen-Effekte des Probenmaterials wirken sich bei diesem Meßverfahren nicht aus. Die Mikrowelle durchdringt die Probe in der aktiven Zone und wird durch die Materialfeuchtigkeit des gesamten betroffenen Proben-Querschnitts in auswertbarer Weise beeinflußt. Damit scheiden systematische Meßfehler aus, wie sie etwa bei Reflexion-Meßverfahren auftreten. Bei Meßverfahren, wo eine an der Proben-Oberfläche reflektierte Mikro- oder Infrarot-Welle ausgewertet wird, entstehen beträchtliche Meßfehler durch eine Veränderung der Rauhigkeit der Oberfläche, der Farbe, oder durch systematische Abweichungen der Oberflächenfeuchte von der Materialfeuchte wie z. B. durch oberflächliches Austrocknen oder durch Konzentration von Feuchte im Oberflächen-Bereich bei pasteusen Produkten nach Press-Vorgängen.

Das Meßverfahren beruht physikalisch auf der Auswertung der Dipol-Relaxation der Wassermoleküle in einer feuchten Materialprobe. Hierzu wird das Meßgut in das elektromagnetische Feld des Resonators in einer optimalen, dem zu messenden Material angepaßten Form gebracht. Die Transmission eines Resonators hängt bei gegebenen geometrischen Abmessungen entscheidend von der Frequenz ab: Sie zeigt Resonanz-Verhalten. Befindet sich das zu messende Material im elektromagnetischen Feld des Resonators, wird die Resonanz-Frequenz des Resonators $f(0)$ gegenüber jener des leeren Resonators $f(L0)$ verkleinert, während die Halbwerts-Breite $b(0)$ der Resonanzlinie gegenüber jener des leeren Resonators $b(L0)$ vergrößert wird. Diese beiden Effekte sind um so größer, je größer die Feuchte des Materials ist. Die Resonanzfrequenz-Verkleinerung $f(L0)-f(0)$ ist eine direkte Folge der Wellenlängenverkürzung vor allem durch das Wasser in der Probe, die Linienverbreiterung $b(0)-b(L0)$ ist eine direkte Folge der Umwandlung von elektromagnetischer Energie in Wärme durch den Wasseranteil im Meßgut. Auf beide meßtechnisch erfaßbaren Parameter hat aber nicht nur die Material-Feuchte, sondern auch die Packungsdichte des Probenmaterials innerhalb des Feldbereichs des Resonators Einfluß. Bei richtiger Anpassung des Applikators an das Probenmaterial ist es immer möglich, die Einflüsse von Materialfeuchte und Materialdichte getrennt zu erfassen. Damit eignet sich das Verfahren hervorragend, gleichzeitig zwei Meßgrößen unabhängig voneinander zu messen, nämlich

- die Materialfeuchte unabhängig von der Packungsdichte, wenn mittels Referenzmessungen eine Feuchte-Kalibration durchgeführt wurde,
- die Materialdichte unabhängig von der Materialfeuchte, wenn mittels Referenzmessungen eine Dichte-Kalibration durchgeführt wurde.

Die Erfindung wird im folgenden am Beispiel der in den Zeichnungen dargestellten Vorrichtung näher erläutert. Es zeigt

Fig. 1       die Vorrichtung in einer schematischen Darstellung als Schaltbild,

Fig. 2       das Blockschaltbild der Vorrichtung nach Fig. 1,

Fig. 3       das Blockschaltbild der Auswerteinheit nach Fig. 2,

Fig. 3a bis 3f       die Bauelemente der Auswerteinheit nach Fig. 3 in einer schematischen Darstellung,

Fig. 4       die Vorrichtung nach Fig. 1 in einer schematischen Vorderansicht,

Fig. 5a bis 5c       den Meßschrank mit Resonator der Vorrichtung nach Fig. 1 in einer schematischen Seitenansicht, Queransicht und Draufsicht,

Fig. 6       ein Flußdiagramm über den zeitlichen Ablauf der Aufnahme der Resonanzkurve,

Fig. 7a bis 10b       Diagramme mit Beispielen für die Feuchtemessung in einem pasteusen Produkt, in Tabak, in Röstkaffee und in einer feinkörnigen Pulversubstanz,

Fig. 11a       eine schematische Darstellung eines Applikators in einer perspektivischen Ansicht,

Fig. 11b und 11c       schematische Darstellungen der elektrischen Feldlinien zweier Sonden bei einem Applikator nach Fig. 11a,

Fig. 12 bis 14       weitere Ausbildungen eines Applikators in schematischen perspektivischen Ansichten,

Fig. 15 und 16       zwei Kalibrationskurven eines Stoffes gemessen bei zwei Resonanzfrequenzen nach Fig. 11b und 11c, eine schematische Darstellung der Auswertung einer mehrdeutigen Kalibrationskurve wie in Fig. 15,

Fig. 17 bis 18        die Dichteunabhängigkeit bei der Feuchtemessung verschiedener Produkte

Fig. 19        die Trennung von Feuchte- und Dichte-Einfluß auf das Resonanzsignal.

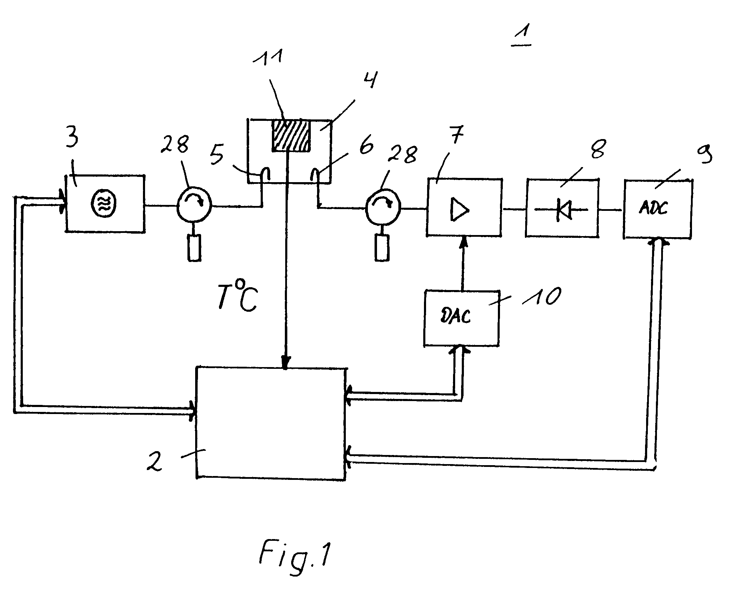

In Fig. 1 ist der grundsätzliche Aufbau einer Vorrichtung 1 zur Feuchtemessung dargestellt. Diese besteht aus einem Applikator 4 in den eine Probe 11 eingeführt werden kann. In dem Applikator 4 sind zwei dem Resonator zugeordnete Ankopplungssonden 5, 6 ausgebildet. Die Ankopplungssonde 5 ist mit einem Mikrowellengenerator 3 verbunden. Die Ankopplungssonde 6 steht über einen Mikrowellenverstärker bzw. -Abschwächer 7 mit einer Detektor-Diode 8 in Verbindung, an die ein Analog/Digital-Wandler 9 angeschlossen ist. Dieser ist mit einem Prozessor 2 verbunden, der über einen Digital/Analog-Wandler 10 mit dem steuerbaren Mikrowellen-Verstärker bzw -Abschwächer 7 in Verbindung steht. Die gewünschte Frequenz des Mikrowellengenerators 3 wird vom Prozessor 2 über eine weitere Verbindung gesteuert. Zwischen dem Mikrowellengenerator 3 und der Ankopplungssonde 5 sowie der Ankopplungssonde 6 und dem Mikrowellen-Verstärker bzw. -Abschwächer 7 ist jeweils zur Entkopplung ein Zirkulator 28 angeordnet. Meßwerte der Temperatur der Probe 11 werden ebenfalls dem Prozessor 2 zugeführt. Der Mikrowellengenerator 3 wird in einem weiten Frequenzbereich betrieben und weist eine PLL-Schaltung auf. Die Auswerteinheit kann z. B. mit einem 68020-Mikroprozessor mit Multitasking- und EPROM-fähigem OS-9-Echtzeit-Betriebssystem ausgerüstet sein. Sie übernimmt sowohl die Kommunikation mit dem Benutzer, die vom Benutzer dirigierte Datenverwaltung, die Durchführung der Meßaufgaben als auch die Weiterverarbeitung der Daten für eine Prozess-Steuerung. Mit dem vom Prozessor 2 digital-abstimmbaren quarzstabilen Mikrowellengenerator 3 wird die Mikrowellen-Leistung variabler Frequenz im notwendigen Frequenzbereich erzeugt. Die vom Prozessor 2 jeweils angeforderte Frequenz wird innerhalb weniger Millisekunden eingestellt (VCO im Rahmen einer PLL-Regelschleife) und dem Applikator 4 zugeführt, der das zu messende Probenmaterial enthält. Dabei erfaßt der Applikator 4 das Meßgut entweder in Form einer Meßprobe oder im "on-line"-Betrieb im Materialstrom.

Das aus dem Applikator 4 kommende Mikrowellen-Signal wird über einen einstellbaren integrierten Mikrowellen-Verstärker bzw. -Abschwächer 7 einer Schottky-Barrier-Detektor-Diode 8 zugeführt, deren NF-Signal mittels eines Analog/Digital-Wandlers 9 vom Prozessor 2 ausgewertet wird.

Der Mikrowellen-Verstärker bzw. -Abschwächer 7 wird vom Prozessor 2 so gesteuert, daß die Detektor-Diode 8 im Falle maximaler Transmittanz des als Resonator ausgebildeten Applikators 4, wenn die Mikrowellenfrequenz mit der aktuellen Resonanzfrequenz des Resonators übereinstimmt, immer die gleiche Mikrowellenleistung zugeführt bekommt.

Auf diese Weise kann die Resonanzlinie des Applikators 4 mit einer gegenüber herkömmlichen Resonator-Meßverfahren deutlich gesteigerten Genauigkeit ausgemessen werden, d. h. Resonanz-Frequenz f(0) und Linienbreite b(0) bestimmt werden. Durch die Prozessor-Steuerung des Mikrowellen-Generators 3 kann mit Hilfe einer PLL-Schaltung die gewünschte Mikrowellen-Frequenz in eng vorgegebenen Frequenz-Schritten exakt eingestellt werden. Durch die Prozessor-Steuerung des vorgeschalteten Mikrowellen-Verstärkers bzw. -Abschwächers 7 kann die Detektor-Diode 8 immer im gleichen Arbeitspunkt maximaler Signalleistung betrieben werden, wodurch der störende Einfluß von Nichtlinearitäten der Diode umgangen wird. Das Diodensignal einer Mikrowellenfrequenz kann weit außerhalb der Resonanz des Applikators 4 erfaßt und als Nullabgleich sowohl der gesamten Meß-Linie wie für die Auswertung der Resonanzlinie herangezogen werden.

Die so im normalen Betrieb für die Bestimmung von Resonanzfrequenz f(0) erreichte Meßgenauigkeit hat den relativen Meßfehler unter den Wert von

$$\Delta f/f(0) < 3 \times 10^{-7}$$

gedrückt.

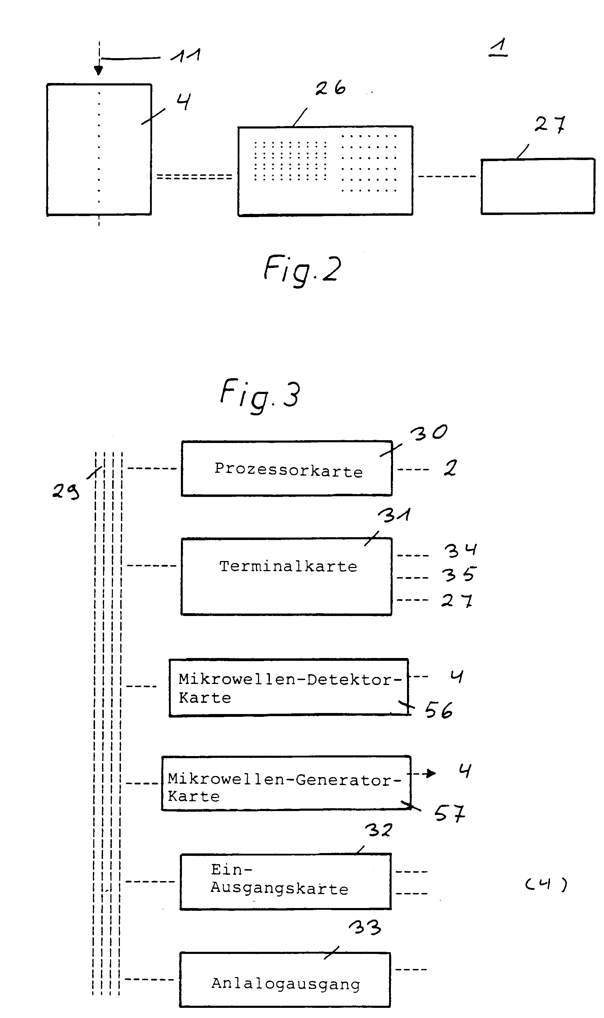

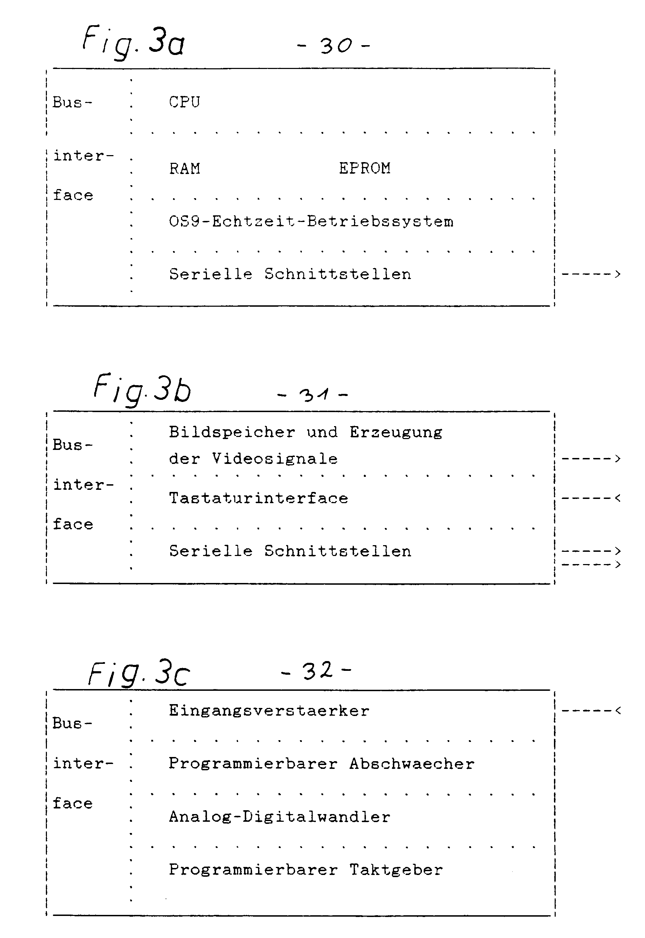

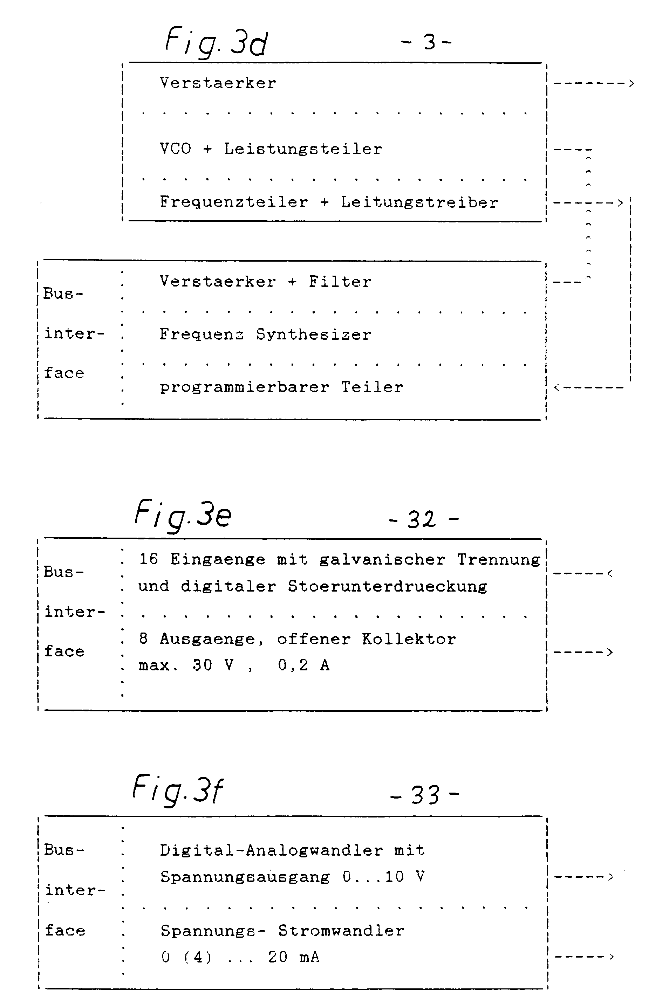

Fig. 2 zeigt ein schematisches Blockschaltbild der Vorrichtung 1 und verdeutlicht, daß diese als Hauptelemente den Applikator 4 für die zu messende Probe 11, eine Auswerteinheit 26 und einen Drucker 27 zur Ausgabe der Meßwerte aufweist. Fig. 3 zeigt ein Blockschaltbild der Auswerteinheit 26 mit einer schematischen Darstellung von deren Bauelementen. An einem Bus 29 ist eine Prozessorkarte 30, eine Terminalkarte 31, der Mikrowellen-Verstärker bzw. -Abschwächer 7, der Mikrowellengenerator 3, eine Ein-Ausgabekarte 32 und ein Analogausgang 33 angeschlossen. Die Prozessorkarte 30 ist mit dem Rechner 2 verbunden. An die Terminalkarte 31 ist ein Display 34 zur Dialogführung und Beobachtung der Messung, eine Tastatur 35 sowie der Drucker 27 angeschlossen. Die Mikrowellen-Detektor-Karte 56 enthält den Mikrowellen-Verstärker bzw. -Abschwächer 7, die Detektor-Diode 8, den Analog/Digital-Wandler 9 und den Digital/Analog-Wandler 10. Die Mikrowellengenerator-Karte 57 weist den Mikrowellengenerator 3 auf, dessen Ausgangssignal zum Applikator 4 geleitet wird. Die Ein-Ausgabekarte 32 dient zur Steuerung des Applikators 4. An den Analogausgang 33 kann eine Fernanzeige für die Prozeßregelung angeschlossen werden. Eine Digital-Schnittstelle erlaubt die Verbindung des Meßgeräts mit einem Prozess-Leitrechner.

In den Figuren 3a bis 3f sind die in der Prozessorkarte 30, der Terminalkarte 31, dem Applikator 4, dem Mikrowellen-Verstärker bzw. -Abschwächer 7, dem Mikrowellengenerator 3, der Ein-Ausgangskarte 32 und dem Analogausgang 33 enthaltenen wesentlichen Bauelemente angegeben.

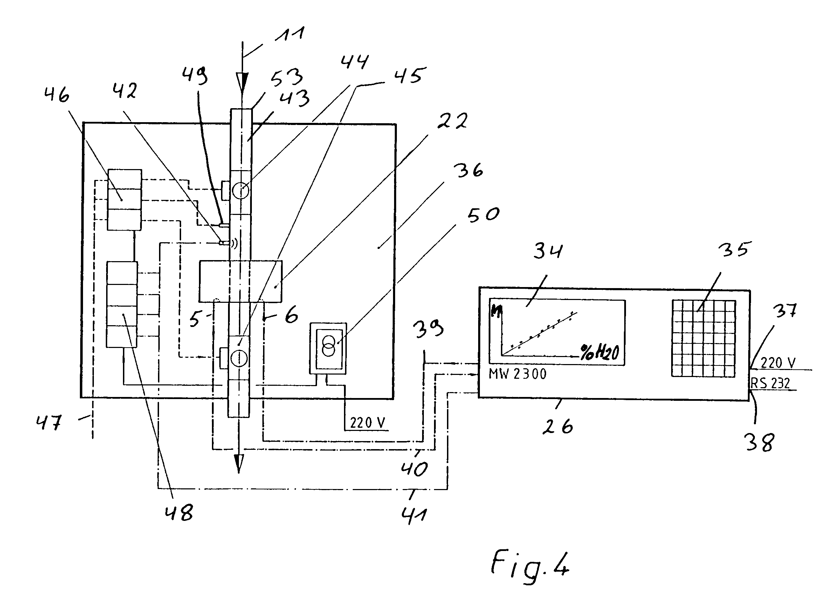

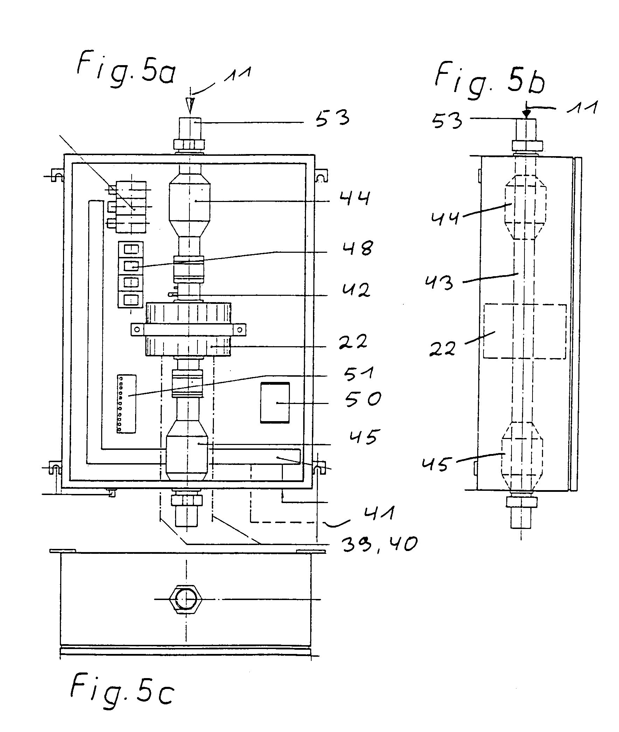

Fig. 4 zeigt die Vorrichtung 1 in einer schematischen Seitenansicht. Die Auswerteinheit 26 mit Display 34 und Tastatur 35 weist einen Netzanschluß 37 und eine Schnittstelle 38 auf, an die z.B. der Drucker 27 angeschlossen werden kann. Mittels Meßleitungen 39, 40 und einer Steuerleitung 41 ist die Auswerteinheit 26 mit den Ankopplungssonden 5, 6 und einer Füllstandssonde 42 verbunden, die im Resonator 22 bzw. in Materialflußrichtung vor diesem im Probenrohr 43 angeordnet sind. Der Resonator 22 und das Probenrohr 43 befinden sich in einem Meßschrank 36. In Materialflußrichtung vor und hinter dem Resonator 22 ist jeweils ein Absperrorgan 44, 45 vorgesehen. Die Absperrorgane 44, 45 werden über Magnetventile 46, die an eine Druckluftleitung 47 angeschlossen sind, mit Druckluft beaufschlagt. Die Magnetventile 46 werden durch Relais 48 gesteuert. Die Relais 48 sind mit der Steuerleitung 41 verbunden und an eine Spannungsversorgung 50 angeschlossen. Ferner ist in dem Probenrohr 43 ein Spühlluftstutzen 49 vorgesehen, der ebenfalls mit den Magnetventilen 46 verbunden ist. Figuren 5a bis 5c zeigen den Meßschrank 36 in verschiedenen Ansichten. Das Relais 48 ist über Klemmen 51 mit der Spannungsversorgung 50 verbunden. Der Materialzulauf 53 ist am oberen Abschnitt des Meßschrankgehäuses 52 angeordnet und dient zum Einfüllen von Meßgut. Am Boden des Meßschrankgehäuses 52 ist ein Druckluftanschluß 54 für die Druckluftleitung 47 vorgesehen. Über ein zweites Absperrorgan 45 wird das Produkt in den Produkt-Hauptstrom zurückgeführt.

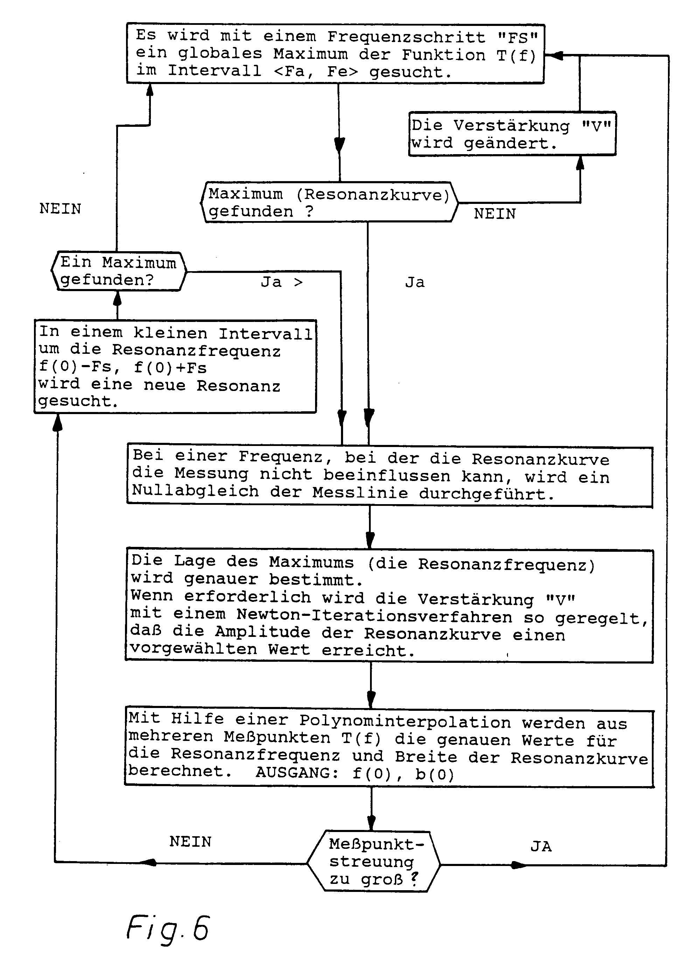

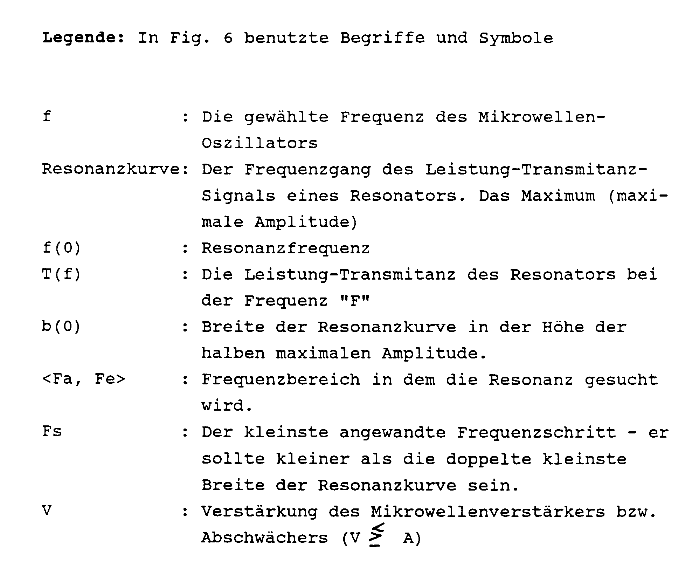

Fig. 6 zeigt in einem Fluß-Diagramm den zeitlichen Ablauf der verschiedenen Rechner-Schritte bei der Steuerung des Prozesses der Aufnahme der Resonanzkurve. Sie verlaufen
- von der Suche des aktuellen Resonanz-Maximums
- über den Nullabgleich weit außerhalb der Resonanz,
- zur Festlegung der notwendigen Mikrowellenverstärkung bzw. -Abschwächung, um den Maximalwert der Resonanzkurve auf einen vorgewählten Wert zu bekommen,
- weiter zur Aufnahme der exakten Meßpunkte der Resonanzkurve,
- bis hin zur rechnerischen Polynom-Interpolation der Resonanz-Linien-Meßwerte, zur genauen Bestimmung von Resonanzfrequenz f(0) und Halbwertsbreite b(0).

Wie bereits erwähnt, sind beide Meßgrößen f(0) und b(0) sowohl von der Materialfeuchtigkeit wie von der Materialdichte abhängig. Der Einfluß beider Größen läßt sich jedoch trennen. Unter der Voraussetzung, daß für die Meßaufgabe der richtig angepaßte Resonator-Typ verwendet wird, können für beliebige dielektrische Stoffe bis in den Bereich sehr hoher Dielektrizitätskonstanten und somit auch hoher Materialfeuchte der Einfluß von Materialfeuchtigkeit und Material-Dichte getrennt werden.

Wenn $\rho$ die Dichte des Materials bezeichnet, das sich im Resonator-Feld befindet und $\Psi_r$ die relative Materialfeuchtigkeit darstellt, dann zeigt die Messung, daß die Breitenänderung b(0)-b(L) und die Frequenzänderung f(0)-f(L) der Resonanzlinie in gegenüber den experimentell zu bestimmenden Bezugsgrößen b(L) und f(L) der gleichen Weise der Dichte des Materials abhängen, unabhängig von der Material-Feuchtigkeit.

Diese gemeinsame Dichtefunktion $X(\rho)$ nimmt mit zunehmender Dichte monoton und im allgemeinen als nichtlineare Funktion von $\rho$ zu.

Wesentlich ist, daß es auf experimentellen Wege möglich ist, die Bezugsgrößen b(L) und f(L) zu bestimmen, so daß eine einzige Funktion $X(\rho)$ angegeben werden kann, die die Dichteabhängigkeit von b(0)-b(L) und f(0)-f(L) in gleicher Weise beschreibt. Damit gilt folgender Produktansatz für die Beziehung zwischen den unmittelbaren Meßgrößen b(0) und f(0) und den zu messenden Stoffgrößen Feuchte $\Psi_r$ und Dichte $\rho$, wobei die empirischen Bezugsgrößen für die Linienbreite b(L) und die Resonanzfrequenz f(L) charakteristisch für einen Resonator-Typ sowie die zu vermessende Materialsorte sind.

$$(1) \qquad b(0) - b(L) = y_b(\Psi_r) * X(\rho)$$

$$(2) \qquad f(L) - f(0) = y_f(\Psi_r) * X(\rho)$$

Eliminiert man durch Division den dichteabhängigen Faktor $X(\rho)$ des Produktansatzes erhält man eine Größe $\Phi$, die vollkommen unabhängig ist von der Dichte $\rho$ des Materials und nur von der Feuchtigkeit $\Psi_r$ abhängt:

$$(3) \qquad \Phi(\Psi_r) = \frac{b(0) - b(L)}{f(L) - f(0)} \frac{y_b(\Psi_r)}{y_f(\Psi_r)}$$

Diese Größe $\Phi$, die aus den primären Meßgrößen b(0), f(0) und den konstanten Bezugsgrößen b(L), f(L) gewonnen wird, ist das eigentliche Mess-Signal der Vorrichtung 1 zur Feuchtemessung. Sie ist nur von der Materialfeuchte abhängig. Wenn sie in der Vorrichtung 1 als Kalibrationskurve abgespeichert vorliegt, kann nach der Bestimmung von $\Phi$ die Materialfeuchte $\Psi_r$ angezeigt werden.

Trägt man die gemessene Linienbreite b(0) der Resonanz-Linie als Funktion der Resonanzfrequenz f(0) bei konstanter Materialfeuchte und variabler Dichte auf, zeigt sich der Einfluß der Dichteschwankung wie folgt. Die Meßwerte von Linenbreite b(0) und Resonanzfrequenz f(0) verändern sich so, daß die Meßpunkte b(0) über f(0) auf einer Geraden liegen, die durch den Punkt mit den empirischen Parametern f(L), b(L) verläuft und deren Steigung alleine von der Feuchtigkeit des Materials abhängt. Nach Gleichung (3) gilt die Geraden-Gleichung

$$(4) \qquad b(0) \; = \; \frac{y_b(\Psi_r)}{y_f(\Psi_r)} \; * \; (f(L) - f(0)) \; + \; b(L)$$

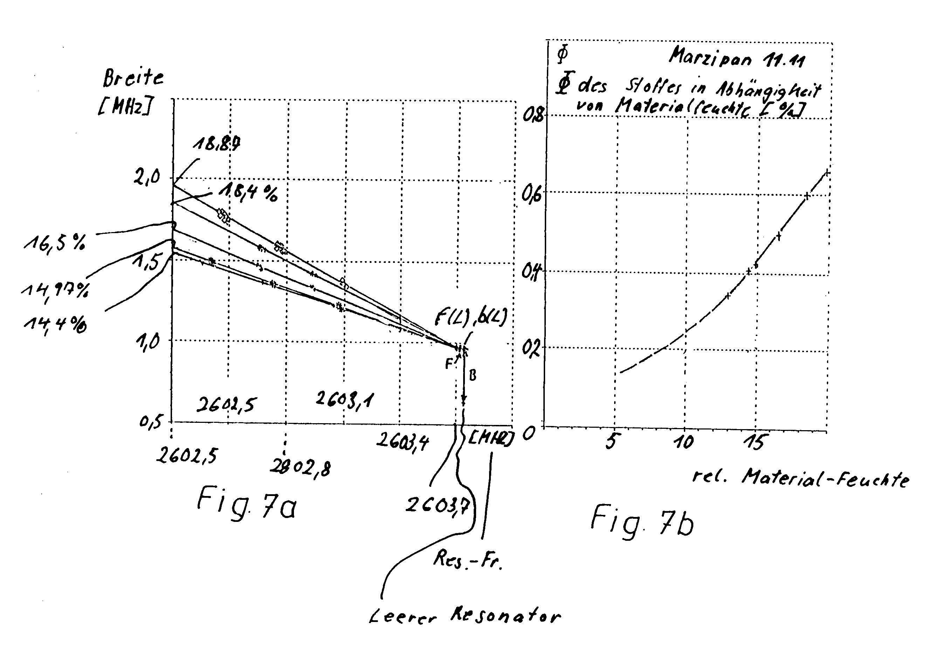

Somit wird auch die Bedeutung der empirischen Parameter b(L) und f(L) deutlich. Sie stellen in der Resonanzfrequenz-Breite-Auftragung den gemeinsamen Schnittpunkt aller durch Dichte-Variation entstandenen Geraden dar, die sich voneinander in der Materialfeuchtigkeit, d. h. in der Geraden-Steigung unterscheiden, wie es z.B. in Fig. 7a dargestellt ist.

Diese beiden empirisch zu bestimmenden Parameter b(L) und f(L) sind für die Verwendung eines bestimmten, für das zu bearbeitende Prozeßfeuchte-Meßproblem optimal angepaßten Resonatortyp durch eine charakteristische Beziehung definiert und werden für den Applikator 4 in der Software des Rechners 2 definiert.

Diese Parameter hängen mit den Kenngrößen der Mikrowellen-Resonanz-Linie des Resonators ohne Meßgut, der Leer-Linienbreite b(L0) und der Leer-Resonanzfrequenz f(L0), über folgende Gleichungen zusammen:

$$(5) \qquad b(L) \; = \; b \, (LO) \; + \; B$$
$$(6) \qquad f(L) \; = \; f(LO) \; + \; F$$

Die beiden Korrekturgrößen B und F für die Abweichung des Dichte-Geraden-Schnittpunktes bei variabler Materialfeuchte von dem Leer-Resonator-Punkt hängen sowohl vom Resonator-Typ wie von der Anpassung des Resonators an das zu messende Material ab.

Insbesondere ändern sich B und F nicht, wenn sich die Leer-Resonator-Daten b(L0) und f(L0) infolge von Veränderungen am Applikator 4 im Verlaufe der Messungen vor Ort verändern sollten, zum Beispiel durch Verschmutzungen des Applikators oder durch thermische Ausdehnung bei Veränderung der Einsatz-Temperatur.

Damit ist es nach den Gleichungen (5) und (6) möglich, den Einfluß von Verschmutzungen des Applikators 4 oder anderer Veränderungen dadurch zu kompensieren, daß einfach die Resonanz-Daten des leeren Resonators b(L0) und f(L0) im normalen Dauerbetrieb der Vorrichtung 1 gemessen werden. Mit den Gleichungen (5) und (6) werden die neuen Bezugsgrößen b(L) und f(L) berechnet und nach Gleichung (3) das Meßsignal aus den direkten Meßgrößen b(0), f(0) bestimmt. Trotz Verschmutzung kann dann mit der gleichen hohen Meßgenauigkeit der Vorrichtung unter Verwendung der alten Kalibrationskurve weitergearbeitet werden.

Entsprechend kann bei Reinigung des Applikators 4 verfahren werden. Durch einfache Messung der Leer-Resonator-Daten ist die Vorrichtung 1 zur Feuchtemessung sofort wieder einsetzbar, ohne daß eine Neu-Kalibration erforderlich ist.

In Fig. 7a und 7b ist am Beispiel eines zur Feuchte-Messung in Marzipan angepaßten Resonators das durch Gleichung (4) beschriebene Verhalten bei Variation der Packungsdichte von Marzipan dargestellt. Deutlich zu erkennen ist, daß die Linienbreite und Resonanz-Frequenz sich längs einer Geraden bewegen, deren Steigung allein von der Materialfeuchtigkeit bestimmt ist. Der durch die Korrekturgrößen B, F gekennzeichnete Schnittpunkt weicht deutlich vom Leer-Resonatorpunkt ab. Daraus ergibt sich die in Fig. 7a und 7b für die Feuchte-Messung in Marzipan typische Kalibrationskurve, wo die nach Gleichung (3) definierte Meßgröße Φ über der Referenzfeuchtigkeit (Trockenschrank-Methode) aufgetragen ist.

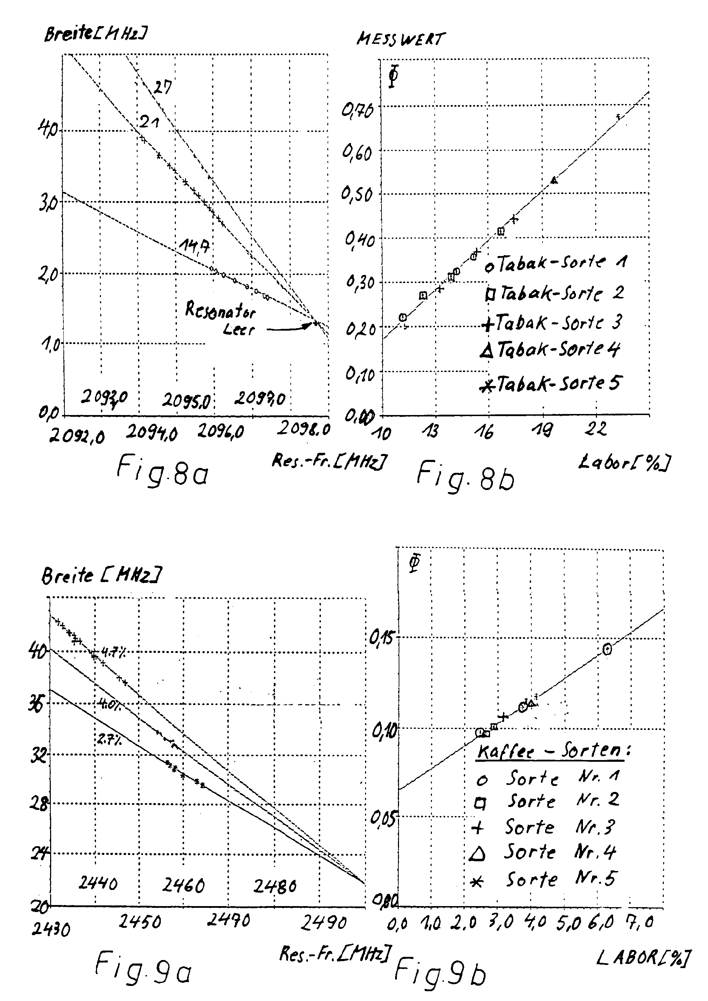

Fig. 8a und 8b zeigen die entsprechenden Meßergebnisse an Tabak im Bereich zwischen 11 und 25 Prozent Materialfeuchte, wobei auch hier die Vergleichsmessungen zur Kalibration der Anlage mit dem Trockenschrank durchgeführt wurden.

Bei dieser Anwendung tritt die Besonderheit zutage, daß die Korrekturgrößen B, F in Gleichung (4) und (5) nahezu Null werden, d. h., die Leerresonatordaten selber in Gleichung (3) die Bezugsgrößen bilden. Weiter sieht man, daß sich die Kalibrationskurve der Meßgröße Φ bei einem Wechsel der Tabak-Sorten mit unterschiedlichem Anbaugebiet und unterschiedlicher Vorbehandlung nur geringfügig ändert.

Noch deutlicher wird diese Sortenunabhängigkeit des Meßsignals bei dem in Fig. 9a und 9b dargestellten Kalibrationskurven-Vergleich von Röst-Kaffeebohnen unterschiedlichster Röstverfahren: Unabhängig von Anbaugebiet und Röstverfahren gilt zur Feuchtemessung die gleiche Kalibrationskurve.

Ursächlich für diese Sortenunabhängigkeit des Meßverfahrens sind die Herabsetzung des Einflusses von Ionen-Leitfähigkeitseffekten durch die Verwendung der Mikrowellen-Meßtechnik sowie die besondere Art der nach Gleichung (3) durchgeführten Signalverarbeitung. Noch vorhandene Sorteneinflüße wirken sich auf Resonanzfrequenz-Verschiebung und Linienverbreiterung ähnlich wie die Materialdichte-Änderung in gleicher Weise aus und werden bei der Division in Gleichung (3) zur Erlangung des Meßsignals herausgekürzt.

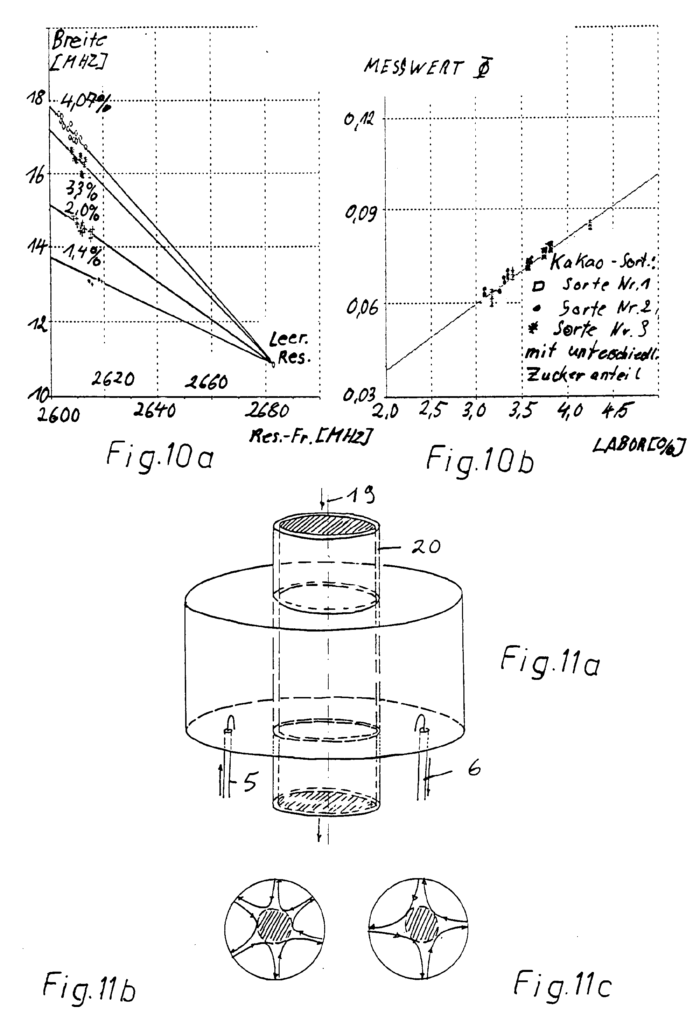

Fig. 10a und 10b zeigt anhand von Feuchtemessungen in Automaten-Kakao-Pulver, daß sich sogar bei deutlicher Variation von Zusatzstoffen im Meßgut, wie hier von Zucker, die Kalibrationskurve des Meßsignals nicht ändert, d. h. nach wie vor mit einer einzigen Kalibrationskurve gearbeitet werden kann.

Die Aufnahme einer Kalibrationskurve kann in einfacher Weise vom Betreiber der Vorrichtung 1 unter Einsatzbedingungen aufgenommen werden, sobald er die Möglichkeit hat, durch eine Referenzmessung die Referenzfeuchte für die Kalibration zu bestimmen (z. B. durch Anwendung der Trockenschrank-Methode). Eine gesonderte Zweitmessung mit Proben definierter Form und Zusammensetzung entfällt ebenso wie der Zwang zur Ausmessung der Mikrowelleneigenschaften des ausgetrockneten Proben-Materials, wie es bei anderen bekannten Mikrowellen-Verfahren notwendig ist.

Damit die in den Gleichungen (1) bis (6) beschriebenen Gesetzmäßigkeiten gelten, muß der Applikator an die jeweilige Feuchtemeßaufgabe angepaßt werden. Hierbei ist folgendes zu beachten:

- Die Ankopplung der zu messende Probe 11 an den Resonator des Applikators 4 hat so zu erfolgen, daß sein Resonanzverhalten nur wenig gestört wird.
- Entelektrisierungseffekte, und damit eine mehr oder weniger vollständige Verringerung der Feuchte-Empfindlichkeit der in Gleichung (3) definierten Meßgröße $\Phi$ lassen sich vermeiden, indem dafür gesorgt wird, daß das elektrische Feld des Resonators beim Übergang in das Material der Probe 11 parallel zur Oberfläche verläuft. Das elektrische Feld darf nicht senkrecht zur Meßmaterial-Oberfläche in die Probe 11 eintreten.

Diese Bedingungen lassen sich durch die besondere Ausbildung des Resonatortyps zur Anpassung an die Meßaufgabe erfüllen.

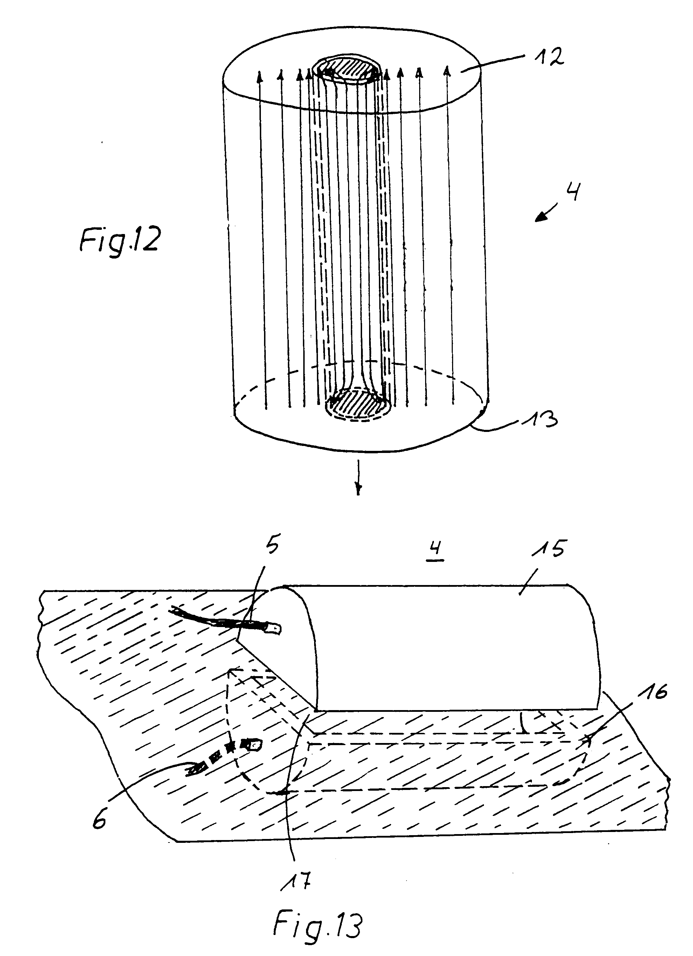

Eine für viele Einsatzfälle zur Materialfeuchtemessung befriedigende Lösung wird dann erreicht, wenn ein kreiszylindrischer Resonatortyp so konstruiert wird, daß in dem in Frage kommenden Frequenzbereich die H311- und H211-Resonanzen angewandt werden können. Dazu muß der Resonator in dem Mittenbereich, in dem die elektrische Feldstärke minimal wird zur Probeneinführung mit einer Öffnung und einer Innenverkleidung versehen werden. Ein solcher Applikator 4 ist in Fig. 11a dargestellt. Koaxial zur Längsachse 19 ist ein Rohr 20 durch den Resonator geführt. Zwischen dem Rohr 20 aus dielektrischem Material und dem Resonatormantel 21 sind einander gegenüberliegend die Ankopplungssonden 5, 6 angeordnet.

Mit einem derartigen Applikator 4 können insbesondere Schüttgüter im Prozeß "on-line" oder mit Probenentnahme vermessen werden. Pasteuse Produkte oder Materialien, die ein Gefäß benötigen, können z.B. in einem becherförmigen Probenhalter in den Resonator eingebracht werden. Eine besondere Anforderung an die Probenform muß nicht gestellt werden. Der maximal mögliche Durchmesser des Rohres 20 ist durch die Forderung begrenzt, daß es in Folge der Wellenlängenverkürzung im Probenmaterial nicht zu elektromagnetischen Abstrahlungen kommt.

Wie in Fig. 11b und 11c zeigen, können im meßbaren Frequenzbereich zwei Resonanzlinien gleichzeitig am selben Produkt ausgemessen werden, die sich um etwa 25 Prozent in ihrer Resonanzfrequenz unterscheiden. Es sind die $H_{311}$-Resonanz mit hexagonaler und die $H_{211}$-Resonanz mit quadratischer Feldsymmetrie, die beide die eingangs erwähnten Bedingungen dadurch erfüllen, daß die zu messende Probe 11 im Randbereich des elektrischen Feldes des Resonators, also in seiner Mitte, angeordnet wird.

Die in den Fig. 8a und 8b an Tabak und in den Fig. 9a und 9b an Röstkaffee bespielhaft dargestellten Meßergebnisse wurden mit einem Applikator gemäß Fig. 11a gewonnen.

Auch eine besondere Konstruktion des kreiszylindrischen Resonators, die im zugänglichen Frequenzbereich die E010-Resonanz aufweist, also die Grund-Resonanz des kreiszylindrischen Resonators, kann für die Feuchtemeßtechnik in besonderen Anwendungsfällen sinnvoll genutzt werden.

Insbesondere bei Materialien mit kleiner Dielektrizitätskonstante, die eine langgestreckte Form haben (z. B. am Zigarettenstrang, Wollfäden, usw.) oder die sich in eine langgestreckte Form bringen lassen (z. B. feinkörnige Schüttgüter) kann ausgenutzt werden, daß das elektrische Feld parallel zur Probenoberfläche verläuft. Für diese Anwendungen ist der in Fig. 12 dargestellte zylinderförmige Applikator 4 besonders gut geeignet. Das Proben-Rohr besteht aus dielektrischem Material. Die Probe 11 wird durch eine Öffnung an der einen Stirnfläche 12 zugeführt und tritt aus der anderen Stirnfläche 13 aus. Hier wird die E010-Resonanz eines kreiszylindrischen Resonators zur Feuchtemessung genutzt.

Die in Fig. 10a und 10b für Kakao-Pulver dargestellten Meßpunkte wurden mit einem Applikator 4 gemäß Fig. 12 gewonnen.

Bei Meßaufgaben, wo die Dielektrizitätskonstante des Materials oder die Materialfeuchtigkeit größer sind (z. B. Feuchtemessung in Marzipan, pasteusen Produkten usw.), kann dieser Resonatortyp verwendet werden, wenn die Probe 11 dünn auf einem dielektrischen Substrat ausgewalzt oder aufgestrichen wird. Dann wird das elektrische Feld beim Eintritt in die Oberfläche der Probe kaum geschwächt. Die in Fig. 7a und 7b für Marzipan dargestellten Meßpunkte wurden auf diese Weise gewonnen.

Für die Feuchtemessung in Folien, Papier- oder Textilbahnen, usw. eignet sich eine kreiszylindrische oder rechteckige Ausbildung eines Applikators 4, der in der E010-Resonanz angeregt werden kann. Dazu wird wie in Fig. 13 der Resonator in Richtung der Wandströme aufgeschnitten, mit einer dielektrischen Innenverkleidung versehen und mechanisch so befestigt, daß die Materialbahn 14 in Richtung des elektrischen Feldes durch den Schlitz 17 des Resonators 4 geführt wird. Mikrowellen-Detektor und -Generator können entweder

EP 0 468 023 B1

mit den unterschiedlichen Resonatorhälften wie abgebildet verbunden sein, oder mit ein und derselben Resonatorhälfte.

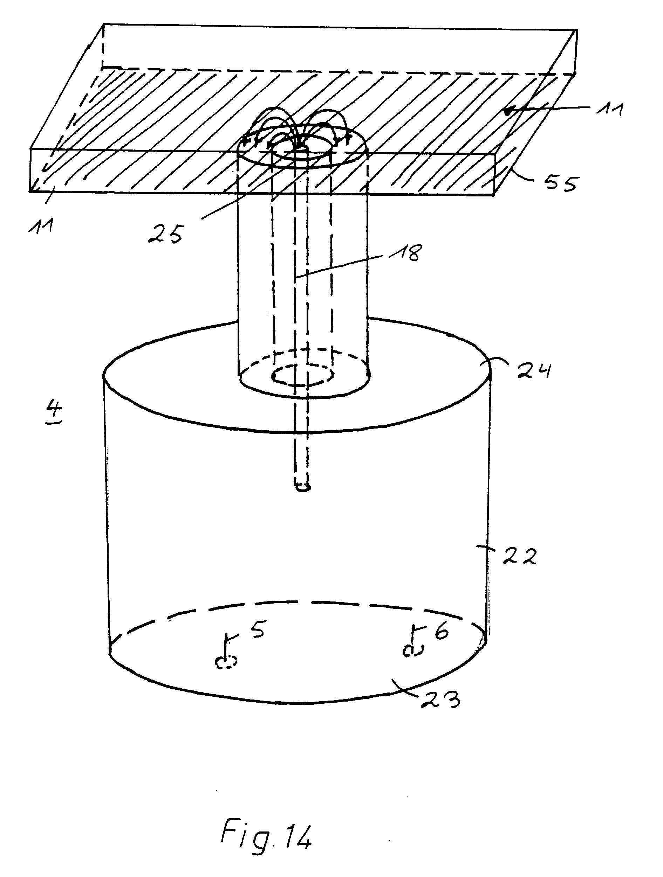

Ein weiterer, für zahlreiche Anwendungsfälle zur Feuchtemessung vor allem in pasteusen Produkten geeigneter Applikator 4 ist in Fig. 14 dargestellt. Auch hier kann die der Gleichung (3) zugrundeliegende Gesetzmäßigkeit bei Einhaltung definierter Bedingungen angewendet werden. Der zylindrische geschlossene Resonator 22 wird wieder im E010-Modus betrieben.

Durch die untere Stirnfläche 23 sind die Ankopplungssonden 5, 6 geführt. Über eine durch die obere Stirnfläche 24 geführte Koaxialleitung 18 der Länge einer viertel Wellenlänge wird ein kleiner Anteil der Mikrowelle im Resonator 22 ausgekoppelt. Am freien Endabschnitt 25 der Koaxialleitung 18 dringt das Streufeld der elektromagnetischen Welle in die Probe 11 ein, die sich auf einem Probenträger 55 befindet. Die Bedingung des parallelen Eindringens der elektrischen Feldstärke in die Probenoberfläche kann durch die Wahl des geeigneten Abstands der Probe von der Öffnung des Koaxial-Resonators erfüllt werden. Zur Messung von $\Phi$ wird die Wechselwirkung des feuchten Materials der Probe 11 mit dem elektrischen Streufeld am offenen Abschluß der Koaxialleitung 18 ausgenutzt.

Der Vorteil dieser Meßmethode ist trotz des relativen kleinen vom elektrischen Feld erfaßten Proben-Bereiches, daß das Probenmaterial nicht durch den Resonator hindurchgeführt werden muß. Vielmehr kann das Material über die aktive Zone des Applikators 4 hinweggeführt werden, wenn der Applikator 4 an Materialführungen, Auflegeplatten, Rohrleitungen, usw. angeflanscht oder eingeschraubt wird.

Die beschriebenen Arten von Applikatoren 4 zeigen, daß das auf Gleichung (3) beruhende dichte- und sortenunabhängige Mikrowellenmeßverfahren für die Materialfeuchte durch die passende Wahl des Applikators 4 auf eine Vielzahl der in der Praxis vorkommenden Aufgabenstellungen angewendet werden kann.

Die meßtechnische Auswertung des nach Gleichung (3) erhaltene Meßsignal $\Phi$ bereitet in einigen Anwendungsfällen das Problem, daß mit zunehmender Materialfeuchte die Größe $\Phi$ nicht wie in den Fig. 7a bis 10b kontinuierlich ansteigt, sondern nach Überschreiten eines Maximums wieder abnimmt. Wenn dieses Maximum aber in einen für den Anwender interessanten Feuchtebereich fällt, würde es bei der Auswertung von $\Phi$ zu möglichen Zweideutigkeiten in der Zuordnung von $\Phi$ und der Materialfeuchte $\Psi_r$ kommen.

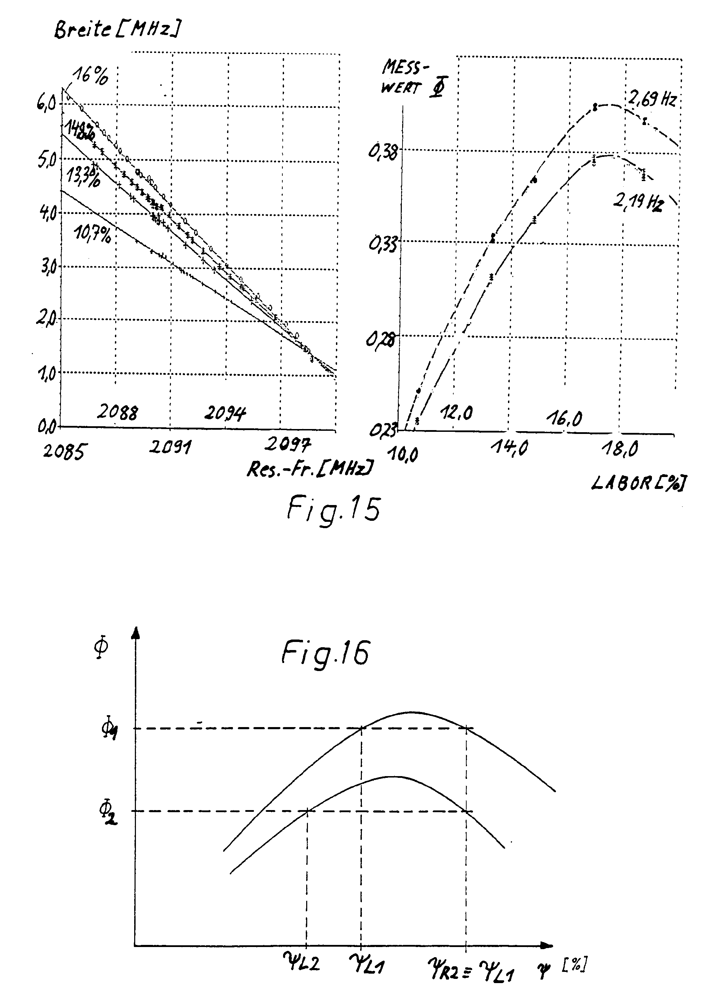

In Fig. 15 ist dies am Beispiel von Parmesan-Käse demonstriert, wo bei einer Feuchte von etwa 17% die Kalibrationskurve ihr Maximum hat, der interessante Feuchtebereich für den Produktionsprozeß aber zwischen 8 und 25% liegt.

Durch die Möglichkeit, gleichzeitig bei zwei und mehr Frequenzen das Resonanzverhalten messen zu können, kann die Vorrichtung 1 unter voller Beibehaltung aller Vorzüge des Problems der Zweideutigkeit überwinden, da die beiden Kalibrationskurven bei den zwei Frequenzen sich voneinander unterscheiden (Fig. 15).

Eine Möglichkeit zur Überwindung der Zweideutigkeit ist in Fig. 16 dargestellt. Danach werden die zwei Meßsignale $\Phi_1$, $\Phi_2$ bei beiden Frequenzen mit der jeweils gültigen Kalibrationskurve links und rechts des Maximums in einen Feuchtewert umgewandelt. Die korrekte Feuchte ergibt sich dann als Mittelwert aus den zwei Feuchtewerten, deren Differenzbetrag minimal ist.

Die Meßgröße $\Phi$ als feuchteunabhängige Größe kann auch ohne Kenntnis der Eigenschaften des leeren Resonators gemessen werden. Dies erfolgt dadurch, daß man die Werte der Halbwertsbreite und Resonanzfrequenz in verschiedenen Dichtezuständen einer Probe im Resonator mißt, diese Werte abspeichert, die Regressionsgerade rechnerisch ermittelt, den Wert der Steigung der Geraden des feuchteabhängigen Meßwerts festhält und über die Kalibrationskurve den Feuchtewert bestimmt.

Der Verfahrensablauf bei dieser zweiten Betriebsart ist wie folgt:

Im Rechner wird eine Kalibrationskurve für die zu messende Materialsorte abgespeichert. Danach wird eine Probe mit gleicher Feuchte aber unterschiedlichen Dichte-Zuständen mit dem Resonator so in Verbindung gebracht, daß das elektrische Feld des Resonators beim Übergang in das Material der Proben allgemein parallel zur Oberfläche verläuft. Für jeden DichteZustand der Probe wird danach die Halbwertsbreite und Resonanzfrequenz gemessen und abgespeichert. Aus den einzelnen Meßwerten wird die Regressionsgerade und damit deren Steigung als Meßsignal $\Phi$ bestimmt. Durch Vergleich der Steigung mit der Kalibrationskurve kann dann der Feuchtewert der Probe ermittelt werden.

Diese zweite Betriebsart der Vorrichtung ermöglicht es somit, auf die Messung des leeren Resonators zu verzichten. Es sind aber Mehrfachmessungen am gleichen Produkt gleicher Feuchte in unterschiedlichen Dichtezuständen erforderlich. Bei vielen pulverförmigen Produkten ist dies sehr gut möglich, aber auch bei Produkten wie Tabak, Käse u.s.w.

Von Vorteil ist dieses Verfahren,

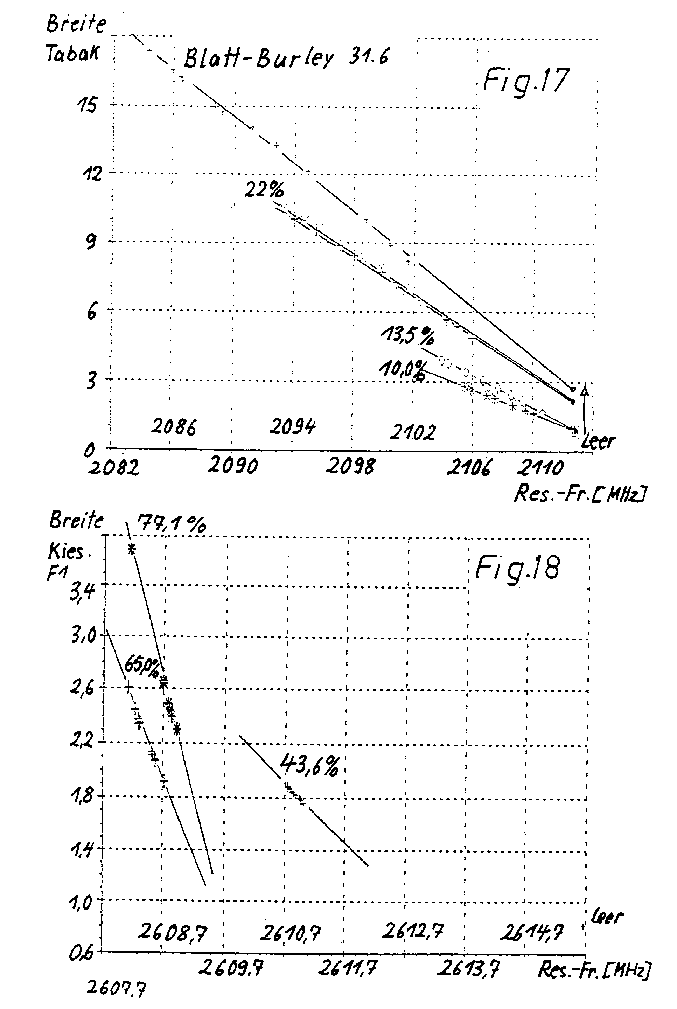

- wenn der Leerzustand des Resonators sich ständig ändert (z. B. sehr starke Verschmutzungen durch das Produkt),
- wenn eine Leerresonanzmessung gar nicht möglich ist, weil das Produkt an einem Strang verläuft,
- wenn die Geraden konstanter Feuchte (oder unterschiedlicher Dichte) sich gar nicht in einem Punkt schneiden (Fig. 17 zeigt dies an Tabak hoher Feuchte, d. h. über 20% und Fig. 18 an Kieselsäure-Preßgut im Feuchtebereich bis 80%)

Fig. 17 zeigt die Dichteunabhängigkeit bei Feuchtemessung in Tabak mit der Mikrowellen-Resonator-Methode:

Die Geraden konstanter Feuchte schneiden sich bei dieser Tabaksorte nur für Feuchtewerte unter 20% in einem Punkt des Breite-Frequenz-Diagramms der Resonanz-Daten.

Mit der normalen Meßmethode des Vergleichs von leerem und vollem Resonator würde also über 20% Feuchte ein Dichteeinfluß feststellbar sein.

Wendet man aber zur Messung der Geradensteigung Φ(ρ) die an Mehrfachmessung von Breite und Frequenz bei verschiedenen Dichtezuständen an, ist die Dichteunabhängigkeit der Feuchtemessung bis zu extremen Tabakfeuchten über 40% gewahrt.

Fig. 18 zeigt als Beispiel für ein Meßgut wie Kieselsäure-Füllstoff (Feuchtebereich 40 - 80%), daß die Geraden konstanter Feuchte im Breite-Frequenz-Diagramm überhaupt keinen gemeinsamen Schnittpunkt mehr haben, so daß mit der einfachen Breite-Frequenz-Messung von leerem und gefülltem Resonator nicht gearbeitet werden kann. Mit der zweiten Betriebsart der Vorrichtung kann dagegen zuverlässig die feuchteabhängige Meßgröße Φ als Steigung der Geraden bestimmt werden. Die Meßgenauigkeit erreicht so +/- 0,2 % bis zu 70% Feuchte.

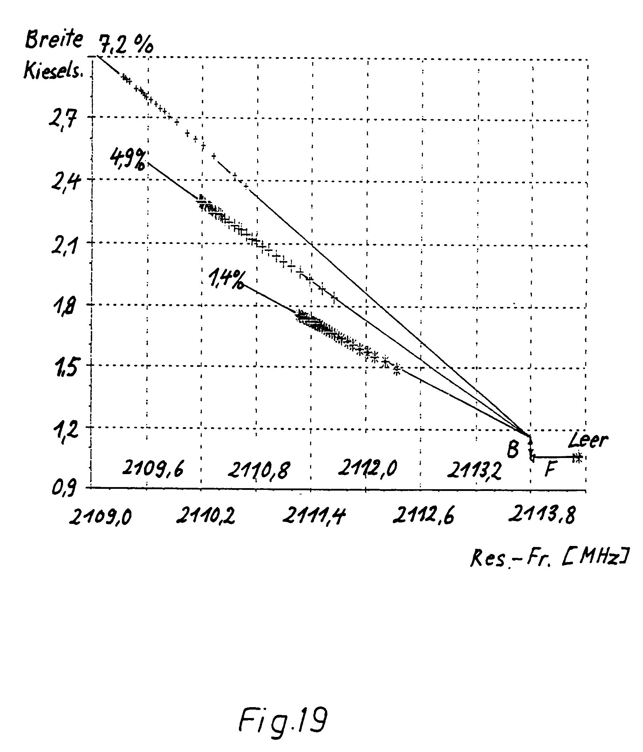

Fig. 19 zeigt am Beispiel eines Resonanz-Frequenz-Breite- Diagramms von Kieselsäure im unteren Feuchtebereich die Trennung von Feuchte- und Dichte- Einfluß auf das Resonanzsignal.

Die Punkte konstanter Feuchte liegen auf einer Geraden mit der Steigung $\Phi = f(\Psi) \neq f(\rho)$. Zwei Methoden eignen sich zur Bestimmung von Φ:

1. Vergleich von Breite, Frequenz der Resonanz von gefülltem und leerem Resonator:

$$\Phi \;=\; \frac{b(0) - (b(L0) + B}{(f(L0) + F) - f(0)}$$

2. Bestimmung der Steigung der Regressionsgeraden durch mehrere Breite-Frequenz-Punkte einer Probe in unterschiedlichen Dichtezuständen.

Dabei ist deutlich zu erkennen, wie mit dieser zweiten Betriebsart, wo die Steigung Φ der Geraden konstanter Feuchte im Breite-Frequenz-Diagramm durch unterschiedliche Kompressionszustände ermittelt wird, das Signal Φ streng dichteunabhängig bleibt.

Die zweite Betriebsart kann auch zur Feuchtemessung nicht komprimierbarer Materialien eingesetzt werden. In diesem Fall erfolgen die Messungen bei unterschiedlichen Füllständen des Probenrohrs. Aus den Meßwerten der unterschiedlichen Füllgradzustände wird dann die Regressionsgerade und deren Steigung bestimmt.

**Patentansprüche**

1. Verfahren zur Messung der Materialfeuchte eines Meßgutes mit Hilfe von Mikrowellen, bei dem durch einen Prozessor gesteuert in einem Mikrowellen-Generator eine elektromagnetische Strahlung variabler Frequenz erzeugt und einem als Resonator ausgebildeten Probenapplikator zugeführt wird, bei dem das aus dem Applikator austretende Mikrowellensignal einer Detektor-Diode zugeführt wird, aus deren Signalen über einen Analog/Digital-Wandler vom Rechner als primäre Meßgrößen b(0) und f(0) ermittelt werden, wobei b(0) die Halbwertsbreite bei der Resonanzfrequenz f(0) des mit einer Meßprobe in Wirkverbindung stehenden Applikators ist, dadurch gekennzeichnet, daß mit dem Resonator eine Materialprobe so in Verbindung gebracht wird, daß das elektrische Feld des Resonators beim Übergang in das Material der Probe allgemein parallel zur Oberfläche verläuft und daß aus den primären Meßgrößen b(0) und f(0) ein Meßsignal Φ gebildet und mit diesem unter Berücksichtigung einer im abgespeicherten Kalibrationskurve für die zu messende Materialsorte die Materialfeuchte ermittelt wird, wobei für das von der Materialfeuchte $\Psi_r$ abhängige Meßsignal $\Phi(\Psi_r)$ die Beziehung gilt

$$\Phi(\Psi_r) \;=\; \frac{b(0) - b(L)}{f(L) - f(O)}$$

mit b(L) und f(L) als konstante material- und applikatorabhängige Bezeugsgrößen, die nach den Beziehungen

$$b(L) \;=\; b(LO) + B$$
$$f(L) \;=\; f(LO) + F$$

ermittelt werden, bei denen b(LO) und f(LO) die Halbwertbreite bzw. Resonanzfrequenz des Applikators ohne Meßgut und B und F applikatortypische Konstanten sind, die abhängig von dem zu vermessenden Material so bestimmt werden, daß das Meß-Signal $\Phi(\Psi_r)$ von der Packungsdichte des Materials unabhängig und nur von der Materialfeuchte abhängig ist und daß durch Messung von b(LO) und f(LO) und Neuberechnung von Φ störende Einflüsse von Resonator-Verschmutzungen und Resonator-Temperaturänderungen kompensiert werden.

2. Verfahren zur Messung der Materialfeuchte eines Meßgutes mit Hilfe von Mikrowellen, bei dem durch einen Prozessor gesteuert in einem Mikrowellen-Generator eine elektromagnetische Strahlung variabler Frequenz erzeugt und einem als Resonator ausgebildeten Probenapplikator zugeführt wird, bei dem das aus dem Applikator austretende Mikrowellensignal einer Detektor-Diode zugeführt wird, aus deren Signalen über einen Analog/Digital-Wandler vom Rechner als primäre Meßgrößen b(0) und f(0) ermittelt werden, wobei b(0) die Halbwertsbreite bei der Resonanzfrequenz f(0) des mit einer Meßprobe in Wirkverbindung stehenden Applikators ist, dadurch gekennzeichnet, daß im Rechner eine Kalibrationskurve für die zu messende Materialsorte abgespeichert wird, daß dann eine Probe mit gleicher Feuchte aber unterschiedlicher Dichtezuständen oder Füllgradzuständen mit dem Resonator so in Verbindung gebracht wird, daß das elektrische Feld des Resonators beim Übergang in das Material der Proben allgemein parallel zur Oberfläche verläuft, und daß dann für jeden Dichtezustand der Probe die Halbwertsbreite und Resonanzfrequenz gemessen und abgespeichert wird, daß dann aus den einzelnen Meßwerten die Regressionsgerade und damit deren Steigung als Meßsignal Φ bestimmt wird und durch Vergleich der Steigung mit Kalibrationskurve der Feuchtewert der Probe ermittelt wird.

3. Verfahren nach Anspruch 1, dadurch gekennzeichnet, daß durch den Prozessor nach dem Einbringen einer Probe in den Wirkbereich des Applikators durch Variation der Frequenz des Mikrowellengenerators das auf die Probe bezogene aktuelle Resonanz-Maximum ermittelt wird, daß dann der Nullabgleich des Sondensignals außerhalb der Resonanz vorgenommen wird, dann der Mikrowellen-Verstärker bzw. -Abschwächer von dem Prozessor so eingestellt wird, daß der Maximalwert der Resonanzkurve einem vorgewählten Wert entspricht und somit Diodennichtlinearitäten sich nicht störend auf die Messung auswirken, dann die exakten Meßpunkte der Resonanzkurve aufgenommen werden und dann aus einer Polynom-Interpolation der Resonanz-Linien-Meßwerte die Resonanzfrequenz f(0) und Halbwertsbreite b(0) ermittelt werden.

4. Verfahren nach Anspruch 1 und 3, dadurch gekennzeichnet, daß die Meßprobe in den Mittelbereichen eines kreiszylindrischen oder Rechteck-Resonators parallel zur Achse des Applikators eingeführt wird, daß in dem Applikator im Falle des kreiszylindrischen Resonators die $E_{010}$-, oder die $H_{211}$- oder die $H_{311}$-Resonanz angeregt wird, so daß das elektrische Feld parallel zur Probenoberfläche in die Probe eintritt.

5. Verfahren nach Anspruch 1 und 3, dadurch gekennzeichnet, daß die Probe auf einem dielektrischen Substrat dünn ausgewalzt oder aufgestrichen oder in Dünnschichtform vorliegend in den Applikator entlang der E-Feld-parallelen Längsachse eines kreiszylindrischen- oder Rechteck-Resonators eingebracht und die Grundresonanz angeregt wird, so daß auch Proben beliebig hoher Feuchte vermessen werden können.

6. Verfahren nach Anspruch 1 bis 3, dadurch gekennzeichnet, daß eine dünne großflächige Probe zwischen den zwei Hälften eines entlang der E-Feld-parallelen Längsachse aufgeschnittenen kreiszylindrischen- oder Rechteck-Resonators geführt und die Grundresonanz angeregt wird.

7. Verfahren nach Anspruch 1 bis 3, dadurch gekennzeichnet, daß aus dem geschlossenen kreiszylindrischen- oder Rechteck-Resonators, in dem die Grundresonanz angeregt wird, über eine Koaxialleitung der Länge einer Viertel-Wellenlänge ein kleiner Teil der Mikrowellenleistung im Resonator ausgekoppelt und die Probe so durch das Streufeld der elektromagnetischen Welle am offenen Ende der Koaxialleitung geführt wird, daß die elektrischen Feldlinien parallel zur Probenoberfläche in die Probe eintreten.

8. Verfahren nach Anspruch 1 oder 2, dadurch gekennzeichnet, daß bei einer Zweideutigkeit zwischen dem Meßwert Φ und der Materialfeuchte $\Psi_r$ (Maximum oder Minimum der Kalibrationskurve) die Meßsignale $\Phi_1$ und $\Phi_2$ in zwei Resonanzmoden unterschiedlicher Resonanzfrequenz erfaßt werden und die Materialfeuchte eindeutig so bestimmt wird, daß die aus den Meßwerten $\Phi_1$, $\Phi_2$ errechnete Feuchte einen minimalen Differenzbetrag aufweist.

9. Vorrichtung zur Durchführung des Verfahrens nach einem der Ansprüche 1 bis 8, gekennzeichnet durch einen von einem Prozessor (2) digital abstimmbaren Mikrowellengenerator (3) mit variabler Frequenz, der mit einer Ankopplungssonde (5) verbunden ist, die in einem Applikator (4) zur Messung der Materialfeuchte einer Probe (11) angeordnet ist, der eine weitere Ankopplungssonde (6) aufweist, die über einen Mikrowellen-Verstärker bzw. -Abschwächer (7) mit einer Detektor-Diode (8) verbunden ist, deren Signalausgang mit dem Prozessor (2) verbunden ist.

10. Vorrichtung nach Anspruch 9, dadurch gekennzeichnet, daß die Frequenz des Mikrowellengenerators (3) mittels einer quarzstabilisierten PLL-Regelschleife einstellbar ist.

11. Vorrichtung nach Anspruch 9, dadurch gekennzeichnet, daß der Applikator (4) als kreiszylindrischer- oder Rechteck-Resonator mit mittiger zur Längsachse (19) koaxialer Durchbrechung zur Einführung einer Probe (11) und einem Rohr aus dielektrischem Material zur Probenführung ausgebildet ist.

12. Vorrichtung nach Anspruch 9, dadurch gekennzeichnet, daß der Applikator (4) aus einem geschlossenen zylindrischen- oder Rechteck-Resonator (22) besteht, durch dessen eine Stirnfläche (23) die Ankopplungssonden (5, 6) und durch dessen andere Stirnfläche (24) mittig eine Koaxialleitung (18) geführt ist, deren freier Endabschnitt (25) im Bereich der Führung für die Probe (11) angeordnet ist.

13. Vorrichtung nach Anspruch 9 bis 12, gekennzeichnet durch einen Meßschrank (36) mit als Resonator (22) ausgebildetem Applikator (4) und einer Auswerteinheit (26), zwischen denen zwei Meßleitungen (39, 40) und eine Steuer leitung (41) angeordnet ist, wobei die Auswerteinheit (26) ein Display (34), eine Tastatur (35), eine Schnittstelle (38) zum Anschluß von Peripheriegeräten, einen Netzanschluß (37) und einen Bus (29) für eine Prozessorkarte (30), eine Terminalkarte (31), eine Mikrowellen-Detektor-Karte (56), eine Mikrowellengenerator-Karte (57), eine Ein-Ausgangskarte (32) und einen Analogausgang (33) aufweist.

14. Vorrichtung nach Anspruch 13, dadurch gekennzeichnet, daß die Steuerleitung (41) mit einer im Probenrohr (43) angeordneten Füllstandssonde (42) und einem Relais (48) für Magnetventile (46) von zwei Absperrorganen (44, 45) verbunden ist, die in dem Probenrohr (43) beidseitig des Resonators (22) angeordnet sind und über die Magnetventile (46) mit einer Druckluft- oder Hydraulikleitung (47) verbunden sind.

## Claims

1. Method for measuring the moisture content of a test sample of a substance with the aid of microwaves, in which electromagnetic radiation of variable frequency is produced by a processor controlled inside a microwave generator and fed to a sample test station configured as a resonator, in which the microwave signal emitted from the test station is fed to a detector diode, from which signals are produced in the form of primary signals b(0) and f(0) via an analogue-to-digital converter of a computer, where b(0) is the half-value width at resonance frequency f(0) of the test station actively coupled to a test sample, characterised in that a test sample of the substance is coupled with the resonator in such a way that the electric field of the resonator runs essentially parallel to the surface of the substance as it penetrates the test sample and that a measurement signal $\Phi$ is derived from the primary measurement values b(0) and f(0) and the moisture content is ascertained using this signal and a calibration curve for the substance type to be measured stored in the memory, the following relationship being applicable to the measurement signal $\Phi(\Psi_r)$ which is dependent on the moisture content of the substance $\Psi_r$

$$\Phi(\Psi_r) = \frac{b(0) - b(L)}{f(L) - f(0)}$$

where b(L) and f(L) are constant reference values relating to the substance and test station produced by the relationships

$$b(L) = b(L0) + B$$
$$f(L) = f(L0) + F$$

where b(L0) and f(L0) are the half-value width and resonance frequency of the test station without a test sample and B and F are constants relating to the test station type, which are determined in relation to the substance to be measured in such a way that the measurement signal $\Phi(\Psi_r)$ is not dependent on the density concentration of the substance and depends only on the moisture content and that by measuring b(L0) and f(L0) and recalculating $\Phi$, distorting influences caused by resonator soiling and changes in resonator temperature are compensated.

2. Method for measuring the moisture content of a test sample of a substance with the aid of microwaves, in which electromagnetic radiation of variable frequency is produced by a processor controlled inside a microwave generator and fed to a sample test station configured as a resonator, in which the microwave signal emitted from the test station is fed to a detector diode, from which signals are produced in the form of primary signals b(0) and f(0) via an analogue-to-digital converter of a computer, where b(0) is the half-value width at resonance frequency f(0) of the test station actively coupled to a test sample, characterised in that a calibration curve for the substance type to be measured is stored in the computer, that a test sample having constant moisture content but varying states of density or filler states is coupled with the resonator in such a way that the electric field of the resonator runs essentially parallel to the surface of the substance as it penetrates the test sample and that for each density state in the test sample the half-value width and resonance frequency are measured and stored, that the regression straight line curve and hence its gradient as a measurement signal $\Phi$ is then defined using the individual measurement values and the moisture content of the test sample determined by comparing the gradient with the calibration curve.

3. Method as claimed in claim 1, characterised in that after introducing a test sample into the operating range of the test station, by varying the frequency of the microwave generator, the current resonance maximum applied to the test sample is determined by the processor, in that the probe signal outside of the resonance is subjected to offset nulling, the microwave amplifier/attenuator of the processor then being set so that the maximum value of the resonance curve corresponds to a preselected value and the diode non-linearities thus do not have a distorting effect on the measuring process, then the exact measurement points of the resonance curve are plotted and the resonance frequency f(0) and half-value width b(0) are determined by means of a polynomial interpolation of the resonance-line measurement values.

4. Method as claimed in claims 1 and 3, characterised in that the test sample is introduced into the middle portion of a tubular or rectangular resonator parallel to the axis of the test station, in that in the test station, if the resonator is a tubular resonator the E010 resonance, or the H211 or H311 resonance is excited so that the electric field enters the test sample parallel to the sample surface.

5. Method as claimed in claims 1 and 3, characterised in that the test sample is rolled or spread thinly onto a dielectric substrate or if in the form of a thin layer placed in the test station along the longitudinal axis of a tubular or rectangular resonator parallel to the E-field and the base resonance excited so that any test samples of a high moisture content can be measured.

6. A method as claimed in claims 1 to 3, characterised in that a thin test sample having a large surface area is fed between the two halves of a tubular or rectangular resonator that is open along the longitudinal axis parallel to the E-field and the base resonance is excited.

7. A method as claimed in claims 1 to 3, characterised in that from the closed tubular or rectangular resonator in which the base resonance has been excited, a small proportion of the microwave output is decoupled from the resonator via a coaxial line of a quarter wavelength in length and the sample is passed through the radiation field of the electromagnetic wave at the open end of the coaxial line in such a way that the electric field lines penetrate the test sample parallel to the surface of the sample.

8. A method as claimed in claim 1 or 2, characterised in that in the event of any ambiguity between the measurement value $\Phi$ and the moisture content of the substance $\Psi_r$ (maximum or minimum of the calibration curve),the measurement signals $\Phi1$ and $\Phi2$ are registered in two frequency modes of different resonance frequency and the moisture content of the substance is unequivocally defined so that the moisture reading calculated from the measurement values $\Phi$, $\Phi2$ has a minimal differential.

9. A device for implementing the method as claimed in one of claims 1 to 8, characterised by a microwave generator (3) with variable frequency that may be digitally set by means of a processor (2), this generator being connected to a coupling probe (5) arranged in a test station (4) for measuring the moisture content of a test sample (11) and having an additional coupling probe (6) connected via a microwave amplifier or reducer (7) to a detector diode (8), whose signal output is connected to the processor (2).

10. A device as claimed in claim 9, characterised in that the frequency of the microwave generator (3) can be adjusted by means of a quartz-stable PLL-control loop.

**11.** A device as claimed in claim 9, characterised in that the test station (4) is constructed as a tubular or rectangular resonator with a central orifice coaxial with the longitudinal axis (19) for inserting a test sample (11) and a tube of dielectric material for conducting tests.

**12.** A device as claimed in claim 9, characterised in that the test station (4) consists of a closed cylindrical or rectangular resonator (22), through one end face (23) of which the coupling probes (5, 6) are fed and through the other end face (24) of which a coaxial line (18) is fed, the free end portion (25) thereof being arranged in the area in which the test sample is introduced.

**13.** A device as claimed in claims 9 to 12, characterised by a measuring cabinet (36) with a test station (4) constructed as a resonator (22) and a plotter (26), between which two measurement lines (39, 40) and a control line (41) are arranged, whereby the plotter (26) has a display (34), a keyboard (35), an interface (38) for connecting peripheral devices, a network terminal (37) and a bus (29) for a processor card (30), a terminal card (31), a microwave detector card (56), a microwave generator card (57), an input-output card (32) and an analogue output (33).

**14.** A device as claimed in claim 13, characterised in that the control line (41) is connected to an occupied state probe (42) arranged in the sampling tube (43) and a relay (48) for magnet valves (46) of two locking elements (44, 45) arranged inside the sampling tube (43) on either side of the resonator (22) and connected via the magnet valves (46) to a pressurised air or hydraulic line (47).

**Revendications**

**1.** Procédé de mesure de l'humidité d'un matériau à mesurer à l'aide de micro-ondes, pour lequel un rayonnement électromagnétique à fréquence variable, commandé par un microprocesseur est produit dans un générateur à microondes et amené à un applicateur d'échantillons se présentant sous la forme d'un résonateur et pour lequel le signal hyperfréquence sortant de l'applicateur est amené à une diode détectrice à partir de laquelle des signaux sont détectés par l'ordinateur par l'intermédiaire d'un convertisseur analogique-numérique, en tant que grandeurs primaires mesurées b(0) et f(0), b(0) étant la largeur de valeur moyenne pour la fréquence de résonance f(0) de l'applicateur qui est en relation active avec un échantillon pour essai, caractérisé en ce qu'un échantillon de matériau est amené de telle sorte en communication avec le résonateur que le champ électrique du résonateur est généralement parallèle à la surface lors de son passage dans le matériau de l'échantillon, en ce qu'un signal de mesure $\Phi$ est formé à partir des grandeurs mesurées b(0) et f(0) et en ce que l'humidité du matériau est détectée avec celui-ci en tenant compte d'une courbe de calibrage mémorisée, pour la sorte de matériau à mesurer, le rapport

$$\Phi(\Psi_r) = \frac{b(0) - b(L)}{f(L) - f(0)}$$

valant pour le signal de mesure $\Phi(\Psi_r)$ dépendant de l'humidité du matériau $\Psi_r$, avec b(L) et f(L) en tant que quantités de référence constantes dépendantes du matériau et de l'applicateur, quantités de référence qui sont déterminées d'après les rapports

$$b\,(L) = b\,(L0) + B$$
$$f\,(L) = f\,(L0) + F$$

pour lesquels b (L0) et f (L0) correspondent à la largeur de valeur moyenne et à la fréquence de résonance de l'applicateur sans matériau et B et F étant des constantes typiques de l'applicateur et qui sont déterminées en fonction du matériau à mesurer de façon à ce que le signal de mesure $\Phi(\Psi_r)$ soit indépendant de la densité de tassement du matériau et ne dépende que de l'humidité du matériau et que des influences parasites d'encrassement du résonateur et des modifications de sa température soient compensées par mesurage de b (L0) et f (L0) et nouveau calcul de $\Phi$.

**2.** Procédé de mesure de l'humidité d'un matériau à mesurer à l'aide de microondes, pour lequel un rayonnement électromagnétique à fréquence variable, commandé par un microprocesseur est produit dans un générateur à microondes et amené à un applicateur d'échantillons se présentant sous la forme d'un résonateur et pour lequel le signal hyperfréquence sortant de l'applicateur est amené à une diode détectrice à partir de laquelle des signaux sont détectés par l'ordinateur par l'intermédiaire d'un convertisseur analogique-numérique, en tant que grandeurs primaires mesurées b(0) et f(0), b(0) étant la largeur de valeur moyenne pour la fréquence de résonance f(0) de l'applicateur qui est en relation active avec un échantillon pour essai, caractérisé en ce qu'une courbe de calibrage pour la sorte de matériau à mesurer est mémorisée dans l'ordinateur, en ce que, par la suite, un échantillon présentant la même humidité mais des états de masse volumique ou de densité différents est amené de telle sorte en communication avec le résonateur que le champ électrique du résonateur est généralement parallèle à la surface lors de son passage dans le matériau des échantillons, en ce qu'ensuite, pour chaque état de masse volumique de l'échantillon, la largeur de valeur moyenne et la fréquence de résonance sont mesurées et mises en mémoire, puis en ce que la droite de régression et donc sa pente sont déterminées à partir des valeurs de mesure séparées en tant que signal de mesure $\Phi$ et la valeur d'humidité de l'échantillon est déterminée en comparant la pente avec la courbe de calibrage.

3. Procédé selon la revendication 1, caractérisé en ce que l'actuel maximum de résonance relatif à l'échantillon est déterminé par le microprocesseur après l'introduction d'un échantillon dans la zone active de l'applicateur, par variation de la fréquence du générateur hyperfréquence, puis en ce que la compensation à zéro du signal de la sonde est effectuée en dehors de la résonance, en ce que la valeur maximale de la courbe de résonance correspond à une valeur présélectionnée et que, donc, les non-linéarités de la diode ne se répercutent pas de façon gênante sur le mesurage, puis en ce que les points de mesure exacts de la courbe de résonance sont relevés et en ce que la fréquence de résonance $f(0)$ et la largeur de valeur moyenne $b(0)$ sont déterminées à partir d'une interpolation de polynôme des valeurs de mesure de lignes de résonance.

4. Procédé selon les revendications 1 et 3, caractérisé en ce que l'échantillon de mesure est introduit dans les zones médianes d'un résonateur cylindrique circulaire ou rectangulaire, en ce que, la résonance $E_{010}$- ou $H_{211}$- ou $H_{311}$- est stimulée dans l'applicateur, dans le cas du résonateur cylindrique circulaire de sorte que le champ électrique pénètre dans l'échantillon parallèlement à sa surface.

5. Procédé selon les revendications 1 et 3, caractérisé en ce que l'échantillon est feuilleté ou enduit sur un substrat diélectrique ou introduit sous forme de couche mince dans l'applicateur le long de l'axe longitudinal d'un résonateur cylindrique circulaire ou rectangulaire, axe longitudinal parallèle au champ E et en ce que la résonance fondamentale est stimulée de façon à ce que des échantillons présentant n'importe quel degré d'humidité puissent être mesurés.

6. Procédé selon les revendications 1 à 3, caractérisé en ce qu'un mince échantillon à grande surface est guidé entre les deux moitiés d'un résonateur cylindrique circulaire ou rectangulaire, ouvert par coupure le long de l'axe longitudinal parallèle au champ E et en ce que la résonance fondamentale est stimulée.

7. Procédé selon les revendications 1 à 3, caractérisé en ce qu'une petite partie des microondes est captée dans le résonateur en provenant du résonateur cylindrique circulaire ou rectangulaire fermé dans lequel est stimulée la résonance fondamentale, par l'intermédiaire d'une ligne coaxiale d'une longueur en quart d'onde et en ce que l'échantillon est guidé de telle sorte dans le champ de dispersion de l'onde électromagnétique, à l'extrémité ouverte de la ligne coaxiale, que les lignes de champ électrique pénètrent dans l'échantillon parallèlement à la surface de celui-ci.

8. Procédé selon les revendications 1 ou 2, caractérisé en ce que, dans le cas d'une ambiguïté entre la valeur de mesure et l'humidité du matériau $\Psi_r$ (maximum ou minimum de la courbe de calibrage) les signaux de mesure $\Phi 1$ et $\Phi 2$ sont détectés dans deux modes de résonance de fréquence de résonance différents et en ce que l'humidité du matériau est nettement déterminée de façon à ce que l'humidité calculée à partir des valeurs de mesure $\Phi 1$, $\Phi 2$ présente une valeur différentielle minimale.

9. Dispositif pour la mise en oeuvre du procédé selon l'une des revendications 1 à 8, caractérisé par un générateur hyperfréquence (3) à fréquence variable et accordable numériquement par un microprocesseur (2), générateur qui est relié à une sonde d'accouplement (5) qui est disposée dans un applicateur (4) pour mesurer l'humidité du matériau d'un échantillon (11), applicateur qui présente une sonde d'accouplement supplémentaire (6) qui est reliée, par l'intermédiaire d'un amplificateur ou d'un amortisseur de microondes (7) à une diode détectrice (8) dont la sortie de signaux est reliée au microprocesseur (2).

10. Dispositif selon la revendication 9, caractérisé en ce que la fréquence du générateur hyperfréquence (3) peut se régler au moyen d'une boucle de régulation PLL stabilisée par cristal.

11. Dispositif selon la revendication 9, caractérisé en ce que l'applicateur (4) se présente sous la forme d'un résonateur cylindrique circulaire ou rectangulaire avec une découpure centrale coaxiale à l'axe longitudinal (19) destinée à l'introduction d'un échantillon (11) et un tube en matériau diélectrique pour guider l'échantillon.

12. Dispositif selon la revendication 9, caractérisé en ce que l'applicateur (4) se compose d'un résonateur cylindrique ou rectangulaire fermé (22) à travers une surface frontale (23) duquel sont guidées les sondes d'accouplement (5, 6) et à travers l'autre surface frontale (24) duquel est guidée de façon centrale une ligne coaxiale (18) dont la section terminale libre (25) est disposée dans la zone de guidage de l'échantillon (11).

13. Dispositif selon les revendications 9 à 12, caractérisé par une table de mesure (36) avec un applicateur (4) se présentant sous la forme d'un résonateur (22) et avec une unité d'évaluation (26) entre lesquels sont disposées deux lignes fendues de mesure (39, 40) et une ligne pilote (41), l'unité d'évaluation (26) présentant un affichage (34), un clavier (35), un point d'intersection (38) pour le raccordement d'appareils périphériques, un branchement sur le réseau (37) et un bus (29) pour une carte de microprocesseur (30), une carte de terminal (31), une carte du détecteur de microondes (56), une carte du générateur hyperfréquence (57), une carte entrée-sortie (32) et une sortie analogique (33).

14. Dispositif selon la revendication 13, caractérisé en ce que la ligne pilote (41) est reliée à une sonde de niveau de remplissage (42) disposée dans le tube d'essai (43) et à un relais (48) pour vannes magnétiques (46) de deux organes d'obturation (44, 45) qui sont disposés dans le tube d'essai (43), des deux côtés du résonateur (22), et sont reliés par l'intermédiaire des vannes magnétiques (46) à une conduite d'air comprimé ou à une conduite hydraulique (47).

Fig.1

*11*

*4*

*1*

*26*

*27*

## Fig. 2

## Fig. 3

*29*

| Prozessorkarte | *30* |
| --- | --- |

---- *2*

| Terminalkarte | *31* |
| --- | --- |

---- *34*
---- *35*
---- *27*

| Mikrowellen-Detektor-Karte | *4* |
| --- | --- |

*56*

| Mikrowellen-Generator-Karte | → *4* |
| --- | --- |

*57*

| Ein-Ausgangskarte | *32* |
| --- | --- |

(*4*)

| Anlalogausgang | *33* |
| --- | --- |

## Fig. 3a    - 30 -

| Bus-<br>inter-<br>face | CPU |
| | RAM            EPROM |
| | OS9-Echtzeit-Betriebssystem |
| | Serielle Schnittstellen -----> |

## Fig. 3b    - 31 -

| Bus-<br>inter-<br>face | Bildspeicher und Erzeugung<br>der Videosignale -----> |
| | Tastaturinterface -----< |
| | Serielle Schnittstellen -----><br>-----> |

## Fig. 3c    - 32 -

| Bus-<br>inter-<br>face | Eingangsverstaerker -----< |
| | Programmierbarer Abschwaecher |
| | Analog-Digitalwandler |
| | Programmierbarer Taktgeber |

## Fig. 3d — 3 —

| Verstaerker | - - - - - - > |
| . . . . . . . . . . . . . . . . . . . . . | |
| VCO + Leistungsteiler | - - - - |
| . . . . . . . . . . . . . . . . . . . . | |
| Frequenzteiler + Leitungstreiber | - - - - - > |

| Bus-<br>inter-<br>face | Verstaerker + Filter | - - - - |
| | Frequenz Synthesizer | |
| | programmierbarer Teiler | < - - - - - - |

## Fig. 3e — 32 —

| Bus-<br>inter-<br>face | 16 Eingaenge mit galvanischer Trennung<br>und digitaler Stoerunterdrueckung | - - - - - < |
| | 8 Ausgaenge, offener Kollektor<br>max. 30 V , 0,2 A | - - - - - > |

## Fig. 3f — 33 —

| Bus-<br>inter-<br>face | Digital-Analogwandler mit<br>Spannungsausgang 0...10 V | - - - - - > |
| | Spannungs- Stromwandler<br>0 (4) ... 20 mA | - - - - - > |

20

Fig. 4

Fig.5a

53

44

48

42

22

51
50

45

41

33, 40

Fig. 5b

53

44

43

22

45

Fig.5c

Es wird mit einem Frequenzschritt "FS"
ein globales Maximum der Funktion T(f)
im Intervall <Fa, Fe> gesucht.

Die Verstärkung "V"
wird geändert.

Maximum (Resonanzkurve)
gefunden ?

NEIN

NEIN

Ein Maximum
gefunden?

Ja >

Ja

In einem kleinen Intervall
um die Resonanzfrequenz
f(0)-Fs, f(0)+Fs
wird eine neue Resonanz
gesucht.

Bei einer Frequenz, bei der die Resonanzkurve
die Messung nicht beeinflussen kann, wird ein
Nullabgleich der Messlinie durchgeführt.

Die Lage des Maximums (die Resonanzfrequenz)
wird genauer bestimmt.
Wenn erforderlich wird die Verstärkung "V"
mit einem Newton-Iterationsverfahren so geregelt,
daß die Amplitude der Resonanzkurve einen
vorgewählten Wert erreicht.

Mit Hilfe einer Polynominterpolation werden aus
mehreren Meßpunkten T(f) die genauen Werte für
die Resonanzfrequenz und Breite der Resonanzkurve
berechnet.   AUSGANG: f(0), b(0)

NEIN

Meßpunktstreuung
zu groß ?

JA

Fig. 6

**Legende:** In Fig. 6 benutzte Begriffe und Symbole


f                        : Die gewählte Frequenz des Mikrowellen-
                           Oszillators
Resonanzkurve: Der Frequenzgang des Leistung-Transmitanz-
                           Signals eines Resonators. Das Maximum (maxi-
                           male Amplitude)
f(0)                     : Resonanzfrequenz
T(f)                     : Die Leistung-Transmitanz des Resonators bei
                           der Frequenz "F"
b(0)                     : Breite der Resonanzkurve in der Höhe der
                           halben maximalen Amplitude.
<Fa, Fe>                 : Frequenzbereich in dem die Resonanz gesucht
                           wird.
Fs                       : Der kleinste angewandte Frequenzschritt - er
                           sollte kleiner als die doppelte kleinste
                           Breite der Resonanzkurve sein.
V                        : Verstärkung des Mikrowellenverstärkers bzw.
                           Abschwächers $(V \lessgtr A)$

**Fig. 7a**

Breite [MHz]

18.8.9
2,0
18,4 %
16,5 %
1,5
14,97%
14,4 %
1,0
F(L),b(L)
F
B
0,5

2602,5    2603,1    2603,4    [MHz]

2602,5    2802,8    2603,7

Res.-Fr.

Leerer Resonator

**Fig. 7b**

Φ    Marzipan 11.11
Φ des Stoffes in Abhängigheit
von Material(feuchte [%]
0,8
0,6
0,4
0,2
0

5    10    15

rel. Material-Feuchte

EP 0 468 023 B1

Breite[MHz]

MESSWERT

27

21

4,0

14,7

3,0

2,0

Resonator
Leer

1,0

2093,0    2095,0    2097,0

0,0

2092,0    2094,0    2096,0    2098,0

Res.-Fr.[MHz]

Fig. 8a

0,70

0,60

0,50

0,40

o Tabak-Sorte 1

0,30    □ Tabak-Sorte 2

+ Tabak-Sorte 3

0,20    △ Tabak-Sorte 4

0,10    ✳ Tabak-Sorte 5

0,00

10    13    16    19    22

Labor[%]

Fig. 8b

Breite [MHz]

40    4,7%

4,0%

36

2,7%

32

28

24

2440    2460    2480

20

2430    2450    2490    2490

Res.-Fr.[MHz]

Fig. 9a

0,15

0,10

Kaffee – Sorten:

o    Sorte    Nr. 1

□    Sorte    Nr. 2

0,05    +    Sorte    Nr. 3

△    Sorte    Nr. 4

✳    Sorte    Nr. 5

0,00

0,0  1,0  2,0  3,0  4,0  5,0  6,0  7,0

LABOR[%]

Fig. 9b

26

Fig.10a

Fig.10b

Fig.11a

Fig.11b

Fig.11c

Fig.12

*12*

*4*

*13*

*5*

*4*

*15*

*16*

*6*

*17*

Fig.13

Fig.14

Breite [MHz]

Fig.15

Fig.16

Fig.17

Fig.18

Fig.19